# EUROPEAN PATENT SPECIFICATION

(11) **EP 0 572 664 B1**
(45) Date of publication and mention of the grant of the patent: **29.12.1999**
(21) Application number: 92905094.6
(22) Date of filing: 24.02.1992
(51) Int. Cl.: C12N 15/12, C12P 21/02, C07K 14/00, C12N 5/10

(54) **HUMAN NERVE CELL ADHESION FACTOR L1, PRODUCTION THEREOF, AND GENE CODING FOR THE SAME**
MENSCHLICHER NERVENZELLENADHÄSIONSFAKTOR L1, DESSEN HERSTELLUNG UND FÜR DENSELBEN KODIERENDES GEN
FACTEUR L1 D'ADHESION SUR CELLULE NERVEUSE HUMAINE, ELABORATION DE CE FACTEUR ET GENE CODANT CELUI-CI

(30) Priority: 22.02.1991 JP 2884291; 06.04.1991 JP 7338191; 18.05.1991 JP 11359691
(43) Date of publication of application: 08.12.1993
(73) Proprietor: Chugai Pharmaceutical Co., Ltd., Tokyo 115 (JP)
(72) Inventor: MIURA, Masayuki 1-9-6-207, Nukuiminamicho, Tokyo 184 (JP); KOBAYASHI, Masaaki 307, Mezon Higashifuji, Gotenba-shi Sizuoka 412 (JP); ASO, Hiroaki, Tokyo 202 (JP); UEMURA, Keiichi, Tokyo 181 (JP)
(74) Representative: Davies, Jonathan Mark
(86) International application number: JP9200192
(87) International publication number: WO9214820

(56) References cited:
- NUCLEIC ACIDS RESEARCH. vol. 19, no. 19 , 11 August 1991 , ARLINGTON, VIRGINIA US pages 5395 - 5401 ROSENTHAL A;MACKINNON RN;JONES DS; 'PCR walking from microdissection clone M54 identifies three exons from the human gene for the neural cell adhesion molecule L1 (CAM-L1).'
- GENOMICS vol. 8, no. 1 , September 1990 pages 113 - 118 CHAPMAN VM;KEITZ BT;STEPHENSON DA;MULLINS LJ;MOOS M;SCHACHNER M; 'Linkage of a gene for neural cell adhesion molecule, L1 (CamL1) to the Rsvp region of the mouse X chromosome.'
- Nature, Vol. 334, No. 25, 1988, MARION MOOS et al., "Neural Adhersion Molecule L1 as a Member of the Immunoglobulin Superfamily with binding domains similar to Fibronectin", p. 701-703.
- Genomics, Vol. 7, No. 4, 1990, MALEK DJABALI et al., "The Gene Encoding L1, a Neural Adhersion Molecule of the Immunogloblin Family, is Located on the X Chromosome in Mouse and Man", p. 587-593.
- Biochimica Biophysica Acta, Vol. 1090, No. 2, 1991, KOBAYASHI, M. et al., "Molecular Cloning of Cell Adhesion Molecule L1 from Human Nervous Tissue a Comparison of the Primary Sequences of L1 Molecules of Different Origin", p. 238-240.
- FEBS (Federation of European Biochemical Societies) Letlers, Vol. 289, No. 1, 1991, MIURA, M. et al., "Molecular Cloning of Complementary DNA Encoding the Rat Neural Cell Adhersion Molecule L1 Two L1 Isoforms in the Cytoplasmic Region are Produced by Differential Splicing", p. 91-95.
- Neuroscience Research (Suppl.), Vol. 0, No. 16, 1991, MIURA, M. et al., "Molecular Cloning and Trarsfection Experiments of Rat L1 Gene", p. S80.
- Journal of Neurochemistry, Vol. 56, No. 3, 1991, HARPER, J.R. et al., "Isolation and Sequence of Partial Complementary DNA Clones of Human L1 Homology of Human and Rodent L1 in the Cytoplasmic Region", p. 797-804.
- Neuroscience Leffers, Vol. 82, No. 1, 1987, TACKE, R. et al., "Identification of Complementary DNA clones of the Mouse Neural Cell Adhersion Molecule L1", p. 89-94.
- Journal of Biological Chemistry, Vol. 263, No. 24, 1988, WOLFF, J.M. et al., "A Human Brain Glycoprotein Related to the Mouse Cell Adhersion Molecule L1", p. 11943-11947.
- Hybridoma, Vol. 10, No. 4, 1991, PATEL, K. et al., "The 200-220 Kda Antigen Recognized by Monoclonal Antibody Mab UJ 127. 11 on Neural Tissues and Tumors is the Human L1 Adhersion Molecule", p. 481-492.

## Description

### FIELD OF THE ART

The present invention relates to human neural cell adhesion molecule L1, a process for production thereof, and a gene encoding same.

### BACKGROUND ART

Neural cell adhesion molecule L1 is a glycoprotein having a molecular weight of 200 KD, isolated, at first, from the cerebellum by affinity chromatography using a monoclonal antibody (Rathjen F.G. et al., EMBO J. 3: 1 - 10, 1984), and involved in neuron-neuron cell adhesion (Keilhauner G. et al., Nature 316: 728 - 730, 1985), and it is understood that the cell adhesion is carried out by homophilic binding between L1 molecules (Lemmon V. et al., Neuron 2: 1597 - 1603, 1989).

Cloning of cDNA for the mouse neural cell adhesion molecule L1 and the analysis thereof were carried out by Moos M. et al. (Nature 334: 701 - 703, 1988), and according to the reference, the L1 molecule belongs to the cell adhesion molecules of the immunoglobulin superfamily, has 6 immunoglobulin C₂ domains (including about 50 amino acid residues between two cysteine residues), and has fibronectin type III domains which are extracellular matrix molecules as known as an adhesive molecule, and is a membrane-penetrating type glycoprotein expected to penetrate the membrane at a region consisting of 23 hydrophobic amino acid residues starting with glycine.

A part of cDNA for the C-terminal side of the human neural cell adhesion molecule L1 (corresponding the 3086 - 3783 position of cDNA for mouse L1 gene) was reported by Harper, J.R. et al., J. Neurochem Vol. 56, p797 - 804 (1991), and a very small portion of genomic DNA (corresponding to the 987 - 1110 position of cDNA for mouse L1 gene) was reported by Djabali M. et al., Genomics, 7: 587 - 593 (1990), but entire features of human neural cell adhesion molecule L1 and the gene therefor have not yet been clarified.

Accordingly, the present invention provides human neural cell adhesive molecule L1, a process for production thereof, and a gene coding therefor.

### DISCLOSURE OF THE INVENTION

Accordingly, the present invention provides the human neural cell adhesion molecule L1.

The present invention also provides a gene coding for the human neural cell adhesion molecule L1.

The present invention further provides a process for production of the human neural cell adhesion molecule L1 characterized by culturing a host transformed with an expression vector comprising said gene and recovering the human neural cell adhesion factor L1.

### BRIEF EXPLANATION OF DRAWINGS

Fig. 1 shows a restriction enzyme map of cDNA fragment RLI coding for a part of rat neural cell adhesion molecule L1, and a probe RL1E/Sac, which is a part thereof.

Fig. 2 shows a restriction enzyme map of a cDNA insert coding for human neural cell adhesion molecule L1.

Fig. 3 shows a positional relationship between a cDNA insert of various plasmids and mouse L1 cDNA.

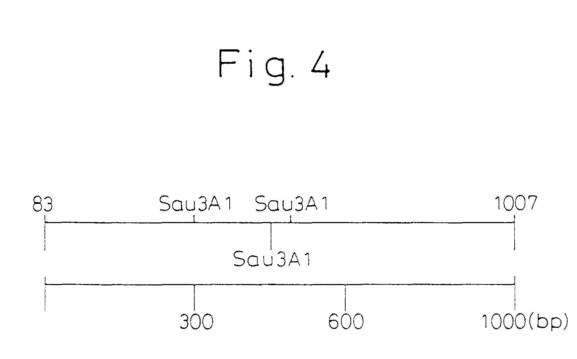

Fig. 4 shows a restriction enzyme map of cDNA insert in plasmid HL1-N4.

Fig. 5 shows a restriction enzyme map (the upper part) of a genomic DNA fragment coding N-terminal region of human neural cell adhesive molecule L1, and a Bam fragment therein (the lower part).

Fig. 6 shows a partial nucleotide sequence in a genomic DNA, comparing with a nucleotide sequence of cDNA for mouse L1.

Fig. 7 shows a partial nucleotide sequence in a genomic DNA, comparing with a nucleotide sequence of cDNA for mouse L1.

Fig. 8 shows a partial nucleotide sequence of cDNA coding for an N-terminal region of human neural cell adhesion molecule L1, comparing with a nucleotide sequence of cDNA for mouse L1.

Fig. 9 shows a partial nucleotide sequence of cDNA coding for an N-terminal region of human neural cell adhesion molecule L1, comparing with a nucleotide sequence of cDNA for mouse L1.

Fig. 10 shows the structure of an expression vector pDE for CHO cells.

### Best Mode for Carrying Out the Invention

According to the present invention, first, a gene coding for human neural cell adhesion molecule L1 is cloned. For this purpose, 4 oligonucleotide primers PL1, PL2, PL3 and PL4 are chemically synthesized on the basis of a nucleotide sequence of cDNA for mouse L1 described in Moos M. et al., Nature Vol. 334, p701 - 703, 1988. On the other hand, mRNA is prepared from the whole brain of rat and mouse. Next, cDNA prepared from the rat mRNA is used as a template and the above-mentioned oligonucleotides PL1 and PL2 are used as primers for a polymerase chain reaction to prepare a cDNA (designated PLC) coding for a part of rat L1. Similarly, a cDNA prepared from the mouse mRNA is used as a template and the above-mentioned oligonucleotides PL3 and PL4 are used as primers to prepare a cDNA (designated PLN) coding for a part of mouse L1.

On the other hand, a cDNA library (HL1065b) (Clon Tech) derived from the human fetal brain is obtained, and this cDNA library is screened by hybridization with a probe prepared by radiolabeling said PLN to obtain a positive clone. An EcoRI fragment containing a cDNA insert in the phage clone is designated as HLN. On the other hand, a vector BSSK-Bam is constructed by replacing Hinc II site in a vector pBluescript SK-(Stratagene) with BamHI site, and the above-mentioned EcoRI fragment HLN is inserted into EcoRI site of the vector to construct plasmid HL1-N4.

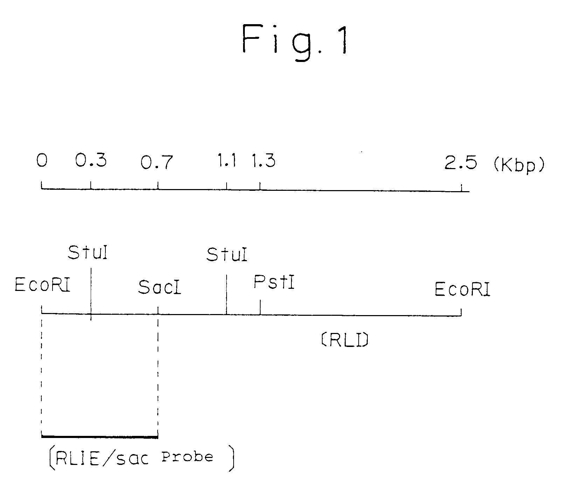

In addition, a rat brain cDNA library is constructed and screened by hybridization with a probe prepared by radiolabeling the above-mentioned PLC to obtain 6 positive clones BSRL1 to BSRL6. Among them the clone BSRL-1 having the longest insert is used to excise therefrom the insert (designated as RL1) as an EcoRI fragment, and a restriction enzyme map is prepared. This map is shown in Fig. 1. Next, the RL1 fragment is further cleaved with Sac I to obtain a DNA fragment (designated as RL1E/Sac) (Fig. 1), which is then radiolabeled to prepare a probe.

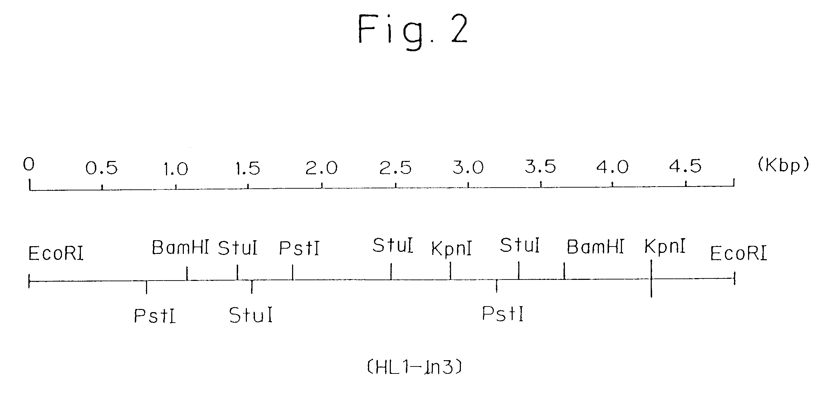

Next, the above-mentioned human cDNA library is screened with this probe RL1E/Sac to obtain 6 positive clones, from which cDNA inserts are excised with EcoRI, and inserted into an EcoRI fragment of said vector BSSK-Bam to obtain plasmids HL1-in1 to HL1-in6 respectively. Among them, a restriction enzyme map of an EcoRI insert in the plasmid HL1-in3 containing the longest cDNA insert (about 5 Kbp) is shown in Fig. 2. This cDNA insert encodes a major portion of human neural cell adhesion molecule L1 except for a part of the N-terminal region. Therefore, cDNA encoding an N-terminal portion is cloned as follows. First, a leukocyte fraction is obtained from human blood, and high molecular weight DNA is extracted therefrom. The DNA is partially digested with a restriction enzyme such as EcoRI to obtain a genomic DNA fragment. This fragment is ligated to the arms of a bacteriophage to obtain a genomic DNA library in E. coli. This library is screened with a probe (PLN) containing a sequence corresponding to 5'-terminal nucleotide sequence 1 to 366 of mouse L1 cDNA to obtain a positive clone.

In this clone, a nucleotide sequence corresponding to the 1 to 366 position in cDNA of mouse L1 exists on a BamHI fragment of about 7 Kbp. This DNA fragment is inserted into BamHI cleavage site in vector pBluescript SK- to obtain HL1-GH. The genomic DNA inserted in this plasmid is sequenced. This genomic DNA contains intron (A) and intron (B) at the positions corresponding to 76 - 77 and 91 - 92 in the nucleotide sequence of cDNA for mouse L1. Therefore, this genomic DNA as such cannot be directly used as a DNA coding for N-terminal region. Accordingly, on the basis of the above nucleotide sequence information, a genomic sequence portion corresponding to the start codon of cDNA for mouse L1 is identified, and an oligonucleotide (PL8) corresponding to a nucleotide sequence of the genomic DNA containing that portion is synthesized. Moreover, an oligonucleotide (PL9) corresponding to the 127 to 148 position in the nucleotide sequence in SEQ ID NO: 2 is synthesized. On the other hand, total RNA preparations are obtained from human adult brain, spinal cord and cauda equia, cDNA is prepared from the combined total RNA, and a cDNA portion corresponding to PL8 to PL9 is amplified by a polymerase chain reaction using the above-mentioned PL8 and PL9 oligonucleotide primers. The product is cleaved with XhoI and StyI to obtain an XhoI-StyI DNA fragment.

On the other hand plasmid HL1-N4 is cleaved with XhoI and StyI and ligated with the above-mentioned XhoI-StyI fragment to construct plasmids, and their nucleotide sequences are determined. Among 5 plasmids, one contained a PCR product of about 230 bp between XhoI-StyI cleavage sites, and another similarly contained a PCR product of about 250 bp. These plasmids are designated as HL1-NPCR3 and HL1-NPCR5. The cDNAs in these plasmids completely encode N-terminal region of human neural cell adhesion molecule L1.

A part of 3'-side of cDNA in the plasmid HL1-NPCR3 and HL1-NPCR5 overlaps with a part of 5'-side of cDNA in the above-mentioned plasmid HL1-in3. Accordingly, HL1-in3 is cleaved with XhoI and SphI to eliminate a 5' side of the coding region, and to the fragment thus obtained, a fragment obtained by cleaving HL1-NPCR3 or HL1-NPCR5 with Xho I and Sph I is joined to obtain a cDNA entirely coding for human neural cell adhesion molecule L1. This complete nucleotide sequence and an amino acid sequence encoded by the nucleotide sequence are shown by SEQ ID NO: 17 and SEQ ID NO: 18 respectively. Accordingly, the present invention relates to human neural cell adhesion factor L1 cDNA coding for the entire amino acid sequences shown by SEQ ID NO: 17 and SEQ ID NO: 18, and further relates to human neural cell adhesion molecule L1 obtained by gene recombination technique using said cDNA.

A region encoded by the nucleotide numbers 3346 to 3414 in SEQ ID NO: 17 and a region encoded by the nucleotide numbers 3361 to 3429 in SEQ ID NO: 18 are expected to be a transmembrane region, and a cDNA wherein this region has been eliminated can be expressed according to a gene recombination technique to obtain a secreted human neural cell adhesion molecule L1. Accordingly, the present invention further provides such a secreted human neural cell adhesion molecule L1. A gene coding for such a secreted human neural adhesion molecule L1 can be obtained starting with the above-mentioned complete DNA according to a conventional means, for example by partial deletion of a coding region by a restriction enzyme cleavage, shortening of DNA by exonuclease, and the like. For example, a secreted L1 molecule can be produced by using a DNA consisting of nucleotide numbers 1 to 3342 in SEQ ID NO: 17 or nucleotide numbers 1 to 3357 in SEQ ID NO: 18, according to the method described in Example 18, and biological activities thereof are confirmed in Example 20.

The present invention further includes various derivatives maintaining at least one of the biological properties of the human neural cell adhesion molecule L1, in addition to the human neural cell adhesion molecule L1 which is an expression product of a gene having the nucleotide sequence shown in SEQ ID NO: 17 or 18. These derivatives include those wherein one or more than one amino acid is deleted from the amino acid sequence of said expression product, those wherein one or more than one amino acid is added to said amino acid sequence, and those wherein one or more than one amino acid is replaced with other amino acids in said amino acid sequence, as well as those containing a combination of at least 2 of the above-mentioned mutations.

Moreover, in addition to those having the nucleotide sequence shown in SEQ ID NO: 17 or 18, the present invention further includes DNAs encoding the amino acid sequence shown in SEQ ID NO: 17 or 18 by other codons. Further, the present invention includes DNAs coding for various derivatives of human neural cell adhesion molecule L1. The present invention still further includes DNAs coding for polypeptides having activity of human neural cell adhesion molecule L1 and capable of hybridize with the nucleotide sequence shown in SEQ ID NO: 17 or 18.

Since a derivative obtained by using a DNA consisting of the nucleotide numbers 1 to 3357 in SEQ ID NO: 18 has biological activities, any derivatives having at least that range of the amino acid sequence and genes coding therefor is included in the present invention as an example of shortened derivatives.

The present invention further includes vectors to which one of the above-mentioned various DNAs has been inserted, for example, plasmids, especially expression plasmids, as well as hosts transformed with said vector, for example animal cells, especially mammalian cells, or eukaryotic or prokaryotic microbial cells. The present invention also provides a process for production of human neural cell adhesion molecule L1 or derivatives thereof using a host transformed with said expression vector.

Namely, a gene, thus obtained, coding for the present human neural cell adhesive molecule L1 or secreted derivatives thereof, or other derivatives thereof can be inserted into an appropriate expression vector, a resulting expression vector can be used to transform an appropriate host, and the transformant thus obtained can be cultured to produce the human neural cell adhesion molecule L1 or a derivative thereof. As hosts for this purpose, prokaryotes such as E. coli, lower eukaryotes such as yeast, higher eukaryotes such as insect or mammalian cells may be used, and for the expression, expression control systems such as promoter, terminator and the like for such a host may be used.

Genes of the present invention thus obtained are used for production of human neural cell adhesion molecule L1 or derivatives thereof according to gene recombination technique. In addition, human neural cell adhesion molecule L1 or derivatives thereof have promise for use as medicaments for treating human neural diseases.

### EXAMPLES

Next, the present invention is explained more definitely by Examples.

### Example 1 Synthesis of oligonucleotide primers

On the basis of a nucleotide sequence of cDNA for mouse L1 described in Moos M. et al., Nature 334, p701 - 703, 1988, oligonucleotides having the following nucleotide sequences were synthesized.

(PL1)5' -CCGGATCCTATTCTAGGGCTACTACAGG-3'          SEQ ID NO: 1

(PL2)5' -GAAGGCCTTTGGCAGCAGCCAGCCATCTCTCAACG-3'          SEQ ID NO: 2

(PL3)5' -GGCAGTTCCTAGCTTATTGCTGGCATAGCAGCGATAGATGCC-3'  SEQ ID NO: 3

(PL4)5' -ATGGTCGTGATGCTGCGGTAC-3'                    SEQ ID NO: 4

The oligonucleotide PL1, PL2, PL3 and PL4 correspond to the 3763 - 3783 position, the 3564 - 3598 position, the 325 - 366 position, and the 1 - 21 position of cDNA nucleotide sequence for mouse L1, respectively. The PL1 has a BamHI cleavage site 5'-CCGGATCC-3' at the 5'-terminus. Each probe was prepared by automatic synthesis using a DNA synthesizer 380A from Applied Biosystems. This method is phosphoamidite method on the basis of the principle described by Curuthers et al. (J. Am. Chem. Soc. 103, p3185, 1981). Since this reaction proceeds in the direction from 3' to 5', 5'-dimethoxytrityl (DMTr)-dG-S(S represents a carrier) is used for the PL1 and PL2, and 5'-DMTr-dC-S was used for the PL3 and PL4 to start the reaction. Next, 5'-DMTr was deprotected and condensed with a phosphoamidite DMTr-dN(dG for PL1, dC for PL2 and PL3, and dA for PL4) previously activated with tetrasol, and free-hydroxyl groups were blocked by acetylation, and then iodine oxidation was carried out to lead to a phosphate. Thereafter, similar condensation was repeated to synthesize the oligonucleotides having the above-mentioned sequences.

The resulting nucleotide PL3 was purified using an Oligonucleotide Purification Cartridges (Applied Biosystems), nucleotides PL1, PL2 and PL4 were recovered in aqueous ammonia, and after that they were purified by 18.5% polyacrylamide gel electrophoresis in the presence of 7M urea, and extracted with 0.2M TBAB (triethylammonium hydrogen carbonate), 1 mM EDTA solution. Next, the extract was absorbed to a DE52 column (Whatman) equilibrated with a solution having the same composition, and eluted with 2M TEAB solution, for purification. Next, DMTr was deprotected in 80% acetic acid and 20% acetonitrile, washings with diethyl ether were repeated 3 times to obtain the desired oligonucleotides.

### Example 2 Purification of rat and mouse brain mRNA and preparation of probes by PCR

### (1) Purification of mRNA

Isolation of mRNA (poly(A)RNA) was carried out on the basis of "Current protocols in molecular biology" (F.M. Ausubel et al., Wiley Interscience, 4.2.3, 1987). To the whole brain (wet weight about 2g) of 2 months old Sprague-Dawley rat, was added 12 ml of GTC solution (50 mM Tris·HCl, pH 7.5, 5M guanidine thiocyanate, 10 mM EDTA, 5% β-mercaptoethanol), and the mixture was thoroughly homogenized in an autoclaved (121°C, 20 minutes) homogenizer, and the homogenate was sucked into a 20 ml syringe having a 23G needle and pushed out. This operation was repeated 5 times. After that 1.2 ml of 5% SSS (sodium sarcosyl sulfate) was added to the homogenate, and the mixture was mixed and incubated at 65°C for 2 minutes. This sample was centrifuged with a Model CD-50SN-type centrifuge (Tomy Seiko) at a room temperature for 10 minutes, at 3000 rpm to obtain a supernatant.

6 ml of CsCl solution (5.7M CsCl, 0.1 mM EDTA) was put into a Polyallomer centrifuge tube, and the supernatant prepared as described above was overlayered. The tube was centrifuged in a Beckman SW40.1 Ti rotor at 30,000 rpm for 15 hours to obtain a pellet which was then suspended in an extraction solution (5 mM EDTA, 0.5% SSS, 5% β-mercaptoethanol), and the suspension was treated with phenol/chloroform (1:1) and with chloroform. To the suspension were added sodium acetate to 0.3M of final concentration and 2.5 volumes of ethanol for ethanol precipitation to obtain total RNA. To purify mRNA from the total RNA, oligo(dT)column chromatography using 3'-terminal poly A chain structure of the mRNA was used. The total RNA was dissolved in an adsorption solution (20 mM Tri·HCl (pH 7.6), 0.5M NaCl, 1mM EDTA, 0.1% SDS (sodium dodecyl sulfate)), and the solution was heated at 65°C for 5 minutes, and passed through and adsorbed on an oligo(dT) cellulose (Pharmacia, PL type 7) column equilibrated with the same adsorption solution.

Next, the resin was washed with 5 column bed volumes of the adsorption solution, and washed again with 2 bed volumes of a washing solution (20 mM Tris·HCl (pH 7.6), 0.2M NaCl, 1 mM EDTA, 0.1% SDS), and elusion was carried out with 2 bed volumes of an elution solution (10 mM Tris·HCl (pH 7.5), 1 mM EDTA, 0.05% SDS). The eluted fraction was treated with phenol/chloroform (1:1) and then with chloroform, and to the fraction were added sodium acetate to 0.3M final concentration and 2.5 volumes of ethanol for ethanol precipitation to obtain a mRNA sample.

Similarly, mRNA was obtained from the whole brain of C57Black mouse (2 months old).

### (2) Synthesis of cDNA by PCR using rat brain mRNA

Polymerase chain reaction (PCR) using the mRNA was carried out using the above-mentioned primers PL1 - PL2, or PL3 - PL4 according to "PCR protocols" (M.A. Innis et al., Academic Press, p21, 1990). The rat brain mRNA (about 2.4 µg) was dissolved in 13 µl of DEPC water (0.1% diethyl-pyrocarbonate), and the solution was heated at 95°C for 5 minutes and rapidly cooled on ice, and to the solution were added 2 µl of 10 × PCR solution (50 mM KCl, 2.5 mM MgCl₂, 20 mM Tris·HCl (pH 8.4), 0.01% bovine serum albumin, × 10), 1 µl of dNTPs solution (dATP, dCTP, dGTP, dTTP, each 10 mM), 1 µl of RNase inhibitor (20 units/µl, Takara Shuzo), 1 µl of random primer (100 pmol/µl, Takara Shuzo) and 1 µl of Moloney Murine leukemia virus transcriptase (200 units/µl, Takara Shuzo), and the mixture was allowed to keep at a room temperature for 10 minutes, and at 42°C for 60 minutes for transcription reaction to synthesize a complementary DNA (cDNA) to the mRNA.

This sample was heated at 95°C for 5 minutes and rapidly cooled on ice, and 80 µl of 1 × PCR solution was added thereon. To this, was added a mixed solution of the synthetic oligonucleotides PL1 and PL2 (each 50 pmol) as primers which provide 3'-OH group and are starting substrates for DNA polymerase reaction, and further 2 units of Taq polymerase (Takara Shuzo) was added. A Zymoreactor AB-1800 (Atto) was used to repeat 40 times a reaction of 94°C - 1.4 minutes, 55°C - 2.4 minutes and 72°C - 3.4 minutes. After the reaction, a part of the product was subjected to 5% acrylamide gel electrophoresis to confirm the presence of the desired cDNA (about 230 base pair (bp)), treated with phenol/chloroform (1:1) and with chloroform. To the product, were added sodium acetate to 0.3M final concentration and 2.5 volumes of ethanol for ethanol precipitation to obtain a pellet, which was then dissolved in 50 µl of TB solution (10 mM Tris·HCl (pH 8.0), 1 mM EDTA) to obtain a PL1-PL2-derived PCR product cDNA (designated as PLC). Next, the PLC was inserted into a vector pBluescript KS+ (see, Stratagene "pBluescript II, EXO/Mung DNA sequencing system, instruction manual") for subcloning.

The primers PL1 and PL2 used for preparation of PLC contained the following sequences:

PL1
↓
5' -GGATCC-3'
3' -CCTAGG-5'
↑

PL2
↓
5'-GAAGGCCT-3'
3'-CTTCCGGA-5'
↑

Since they have recognizing sites for restriction enzymes BamHI or StuI, PLC (0.3 µg) was cleaved with BamHI and StuI (both from Takara Shuzo), and similarly the vector pBluescript KS+ was cleaved with BamHI and HincII which was a blunt-end-providing restriction enzyme.

Both the PLC and pBluescript KS+ were treated with phenol/chloroform (1:1) and with chloroform, and sodium acetate was added to 0.3M final concentration and 2.5 volumes of ethanol for ethanol precipitation to obtain a pellet, which was then dissolved in 15 µl of distilled water. Next, 1 µl of the pBluescript KS+ and 2 µl of the PLC were mixed, and to the mixture were added 1 µl of 10 × ligase solution (0.66M Tris·HCl (pH 7.6), 50 mM MgCl₂, 50 mM dithiothreitol (DTT)), 1 µl of 10 mM ATP, and 1 µl of T4-DNA ligase (Takara Shuzo, 3.4 units/µl). The mixture was filled up to 10 µl with distilled water, and a ligase reaction was carried out at 12°C for 12 hours to construct a plasmid wherein PLC has been inserted into pBluescript KS+. The resulting plasmid is designated BSKS-PLC.

The BSKS-PLC was used to transform E. coli HB101 competent cells (Takara Shuzo), and after culturing a transformant a large amount of the plasmid BSKS-PLC was obtained according to an alkaline denaturation method (Maniatis et al., "Molecular Cloning, Cold Spring Harbor, p368, 1982). After that, the BSKS-PLC was fragmented with BamHI and XhoI (Takara Shuzo) to form a insert DNA, which was then isolated by 1% agarose gel electrophoresis, and PLC was purified using a Geneclean Kit (BIO 101).

### (3) Synthesis of cDNA by PCR using mouse brain mRNA

Similarly to Example 2(2), PCR using mRNA was carried out according to "PCR protocols".

Mouse brain mRNA (about 2.4 µg) was dissolved in 13 µl of DEPC water, the solution was heated at 95°C for 5 minutes and rapidly cooled on ice, and to the solution were added 2 µl of 10 × PCR solution, 1 µl of dNTP solution, 1 µl of RNase inhibitor, 1 µl of random primer and 1 µl of Moloney Murine leukemia virus reverse transcriptase. The mixture was allowed to keep at a room temperature for 10 minutes, and transcriptase reaction was carried out at 42°C for 60 minutes to synthesize a complementary DNA (cDNA) to mRNA. This sample was heated at 95°C for 5 minutes and rapidly cooled on ice, and 80 µl of 1 × PCR solution was added thereon. To this mixture were added a mixed solution of the synthetic oligonucleotide PL3 and PL4 (each 50 pmol) and 2 units of Taq polymerase (Takara Shuzo), and reaction of 94°C - 1.4 minutes, 55°C - 2.4 minutes and 72°C - 3.4 minutes was warried out for 40 cycles.

After the reaction, a portion of the sample was used to confirm the presence of the desired cDNA (about 360 bp) by 5% polyacrylamide gel electrophoresis, and the sample was treated with phenol/chloroform (1:1) and with chloroform, and were added thereon sodium acetate to make the final concentration 0.3M, and 2.5 volumes of ethanol for ethanol precipitation to obtain a pellet. The pellet was then dissolved in 50 µl of TE solution to obtain PL3-PL4-derived PCR product cDNA (designated as PLN). Next, the PLN was subcloned by inserting it into a vector pBluescript KS+.

Since the primers PL3 and PL4 used for PLN have no restriction enzyme cleavage sites, blunt ends were formed by using Mung bean nuclease and DNA polymerase (Klenow fragment). PLN (0.3 µg) was dissolved in 50 µl of Mung Bean solution (30 mM sodium acetate, 50 mM NaCl, 1 mM ZnCl₂, 5% glycerol, pH 5.0), and 1 µl of Mung bean nuclease (15 units/µl, Takara Shuzo) was added thereon. After reaction at 30°C for 30 minutes, the mixture was treated with phenol/chloroform (1:1) and with chloroform, and were added thereon sodium acetate to 0.3M final concentration, and 2.5 volumes of ethanol for ethanol precipitation. Resulting pellet was dissolved in 25 µl of distilled water, and 25 µl of DNA polymerase (Klenow fragment) solution (50 mM Tris·HCl (pH 7.2), 10 mM MgCl₂, 0.1 mM DTT, 1 mM dNTPs) and 1 µl of Klenow fragment (Takara Shuzo) were added thereon to carry out a reaction at 30°C for 30 minutes.

Previously, pBluescript KS+ was cleaved with HincII, and both the PLN and pBluescript KS+ were treated with phenol/chloroform (1:1) and with chloroform, and sodium acetate was added to 0.3M final concentration, and 2.5 volumes of ethanol was added for ethanol precipitation to obtain a pellet, which was then dissolved in 5 µl of distilled water. Next, 1 µl of pBluescript KS+ and 2 µl of PLN were mixed, 1 µl of 10 × ligase solution, 1 µl of 10 mM ATP and 1 µl of T4 DNA ligase were added thereon, and the whole was made up to 10 µl with distilled water. Ligase reaction was carried out at 12°C for 12 hours to construct a plasmid wherein PLN has been inserted into pBluescript, KS+. The resulting plasmid is designated as BSKS-PLN.

The BSKS-PLN was used to transform E. coli HB101 competent cells, and after culturing the transformant, a large amount of plasmid BSKS-PLN was obtained by an alkaline denaturation method. Next, the BSKS-PLN was digested with HincIII and XhoI (both, from Takara Shuzo) to form an inserted DNA, which was then isolated by 1% agarose gel electrophoresis, and PLN was purified by a Geneclean Kit (BIO 101).

### Example 3 Construction of cDNA library

### (1) Construction of rat brain cDNA library

The construction of a rat brain cDNA library is summarized as follows.

Rat brain mRNA was obtained by a method described in Example 2(1), and a single-stranded cDNA was synthesized by reverse transcriptase using oligo(dT)nucleotide as a primer. Next, double-stranded cDNA was prepared using RNase H and DNA polymerase I. Next, blunt ends were formed using T4 DNA polymerase etc., and an EcoRI linker was ligated thereto. The DNA fragment thus obtained was joined to the arm DNA of E. coli phage λ - ZAP vector (Stratagene) having restriction enzyme EcoRI cleavage sites to construct infective recombinant phage DNA. The recombinants were converted to phage particles by in vitro packaging method (T. Maniatis et al., "Molecular Cloning", p286) to finalize the construction of the library.

### (2) Construction of a cDNA library derived from human fetal brain

The cDNA library derived from human fetal brain was that (HL1065b) purchased from Clonetech, and has the following properties.

A mRNA preparation was prepared from a human fetal whole brain after 21 weeks from fertilization, and oligo(dT)-primed cDNA was inserted into the arm vector EcoRI cleavage site of E. coli phage λgtll, according to R.A. Young's method (Proc. Natl. Acad. Sci. U.S.A., 80, p1194, 1983).

### Example 4 Screening of rat brain-derived cDNA library

Plaque hybridization was carried out according to Benton and Davis method (Science, Vol. 196, p180, 1977).

Namely, the PLC obtained in Example 2(2) was radiolabeled with α[³²P]-dATP according to random primer method (Anal. Biochem., 132, 6, 1983). On the other hand, E. coli Y1090^{r-} was infected with phage from the rat brain cDNA library, and phage plaques on an agar plate were transferred to a Biodyne nylon membrane (Pall Biosupport), and the nylon membrane was treated as follows.

DNA was denaturated with a denaturation solution (1.5M NaCl, 0.5M NaOH) for one minute, and the membrane was twice neutralized with a neutralization solution (3M sodium acetate, 0.44% acetic acid) for 5 minutes for each time, dried at a room temperature, and heated at 80°C for 2 hours to fix DNA. Next, after prehybridization at 42°C for 2 hours in a hybridization solution containing 5 × SSPE solution (1 × SSPE = 180 mM NaCl, 10 mM NaH₂PO₄·2H₂O, 1 mM EDTA, pH 7.4), 5 × Denhardt's solution (1 × Denhardt = 0.02% Ficol, 0.02% polyvinyl pyrrolidone, 0.02% bovine serum albumin), 50% formamide, 0.1% SDS and 0.1 mg/ml denatured DNA (salmon testes DNA), bybridization was carried out with a hybridization solution containing a radio-labeled PLC probe (400000 cpm/ml) at 42°C overnight.

Next, the membrane was twice washed with 2 × SSC solution (1 × SSC = 150 mM NaCl, 15 mM sodium citrate, pH 7.0) containing 0.1% SDS at a room temperature for 15 minutes, and further washed with 0.1 × SSC solution containing 0.1% SDS at 50°C for 20 minutes, and detection by autoradiography was carried out. As a result, 6 positive clones were obtained. From these clones, phagemids (BSRL-1 to BSRL-6) were obtained by in vitro excision method (see, Stratagene, instruction manuals, # UC108). Namely, 6 cloned phages were infected to E. coli XL1-blue, and then R408 helper phage (Stratagene) was infected. Since DNA of phage λ-ZAP contains an origin of replication and a termination region from f1 phage, plasmid (pBluescript SK-) wherein cDNA has been inserted into an EcoRI cleavage site was secreted as phagemid by infection of R408 helper phage.

After this reaction was carried out at 37°C for 3 hours, the microbial cells were disrupted by a heating at 70°C for 20 minutes, and was centrifuged at 800 rpm for 5 minutes to collect the supernatant. On the other hand, competent cells were prepared from E. coli XL1-blue by a method described by D. Hanahan (DNA cloning, IRL Press, p109, 1985), and transformed with the phagemid prepared above, and after culturing the transformant, 6 plasmids (BSRL1 to BSRL6) were obtained by an alkaline denaturation method. Among these plasmids, an inserted DNA (designated as RL1) was fragmented from BSRL1 having the longest insert using EcoRI, and a prepared restriction enzyme map is shown in Fig. 1.

### Example 5 Screening of human fetal brain-derived cDNA library (1)

The human fetal brain-derived cDNA library was screened according to the Benton and Davis's method described in Example 4. Namely, E. coli Y1090r⁻ (a recA-mutant of Y1090) was infected with phage from the human fetal brain-derived cDNA library, from an agar medium on which phage plaques were developed, the phage was transferred on a Biodyne nylon membrane, and the nylon membrane was treated by the same procedure as described in Example 4. DNA was denaturated with a denaturation solution for one minute, followed by twece neutralization with a neutralizing solution for 5 minutes, and after drying the membrane at a room temperature, DNA was fixed at 80°C for 2 hours.

Next, prehybridization was carried out in a hybridization solution at 42°C for 2 hours. On the other hand, the PLN obtained from Example 2(3) was radio-labeled by a random primer method to prepare a probe. Next, hybridization was carried out using a hybridization solution containing the probe PLN (400000 cpm/ml) at 42°C overnight. After that, the nylon membrane was twice washed with 2 × SSC containing 0.1% SDS at room temperature for 15 minutes, and further washed with 0.1 × SSC solution containing 0.1% SDS at 50°C for 20 minutes, and detection was carried out by autoradiography. As a result, one positive clone was obtained. E. coli Y1090r⁻ was infected with phage from this clone, and the phage DNA was obtained according to a procedure described by Maniatis et al. (Molecular Cloning, p80, Cold Spring Harbor Laboratory, 1982). The inserted DNA was fragmented with EcoRI, and purified using a Geneclean Kit. This fragment is designated as HLN.

On the other hand, vector pBluescript SK- was cleaved with Hinc II, and to the cleaved plasmid, a BamHI linker (5'-dCCGGATCCGG-3': Takara Shuzo) phosphorylated at its 5'-terminus with T4 polynucleotide kinase (Takara Shuzo) was inserted using T4 DNA ligase to construct a vector BSSK-Bam. Next, the above-mentioned DNA fragment HLN was inserted into BSSK-Bam at EcoRI cleavage site according to a procedure described in Example 2. This plasmid was used to transform E. coli XL1-blue competent cells, and after culturing, plasmid was obtained by an alkaline denaturation method. The plasmid is designated as HL1-N4.

Escherichia coli HB101 [HL1-N4] containing this plasmid was deposited with Fermentation Research Institute Agency of Industrial Science and Technology, as FERM BP-3257 on January 30, 1991, as an international deposition under the Budapest treaty.

### Example 6 Screening of human fetal brain-derived cDNA library (2)

Next, among the BSRL-1 to L6 obtained in Example 4, a DNA fragment was obtained from the BSRL1 having the longest insert by cleavage with EcoRI, and further cleaved with SacI (Takara Shuzo), and a resulting fragment (designated as RL1E/Sac; about 0.7 Kbp, shown in Fig. 1) was purified using a Geneclean Kit. The RL1E/Sac was radio-labeled by a random primer method to prepare a probe, which was used to screen the library to obtain 6 positive clones. E. coli Y1090 was infected with phage from these clones, and phage DNA was obtained according to a method described by Maniatis et al., and the inserted DNA was fragmented with EcoRI. This fragment was inserted into the above-mentioned vector BSSK-Bam at EcoRI cleavage site according to the method described in Example 2, and used to transform E. coli HB101 competent cells, and after culturing, plasmid was obtained by an alkali method. These plasmids are designated as HL1-in1 to 6. Among them, an inserted DNA was fragmented from plasmid HL1-in3 incorporating the longest DNA chain (about 5000 bp) with EcoRI, and a prepared restriction enzyme map is shown in Fig. 2.

Note, Escherichia coli HB101 [HL1-in3] containing this plasmid was deposited with Fermentation Research Institute, Agency of Industrial Science and Technology, as FERM BP-3256 on January 30, 1991, as an international deposition under the Budapest treaty.

### Example 7

For the plasmid HL1-N4 obtained in Example 5 and plasmids HL1-in1 to HL1-in6 obtained in Example 6, both the ends of each clone were sequenced.

Reading of sequence was carried out according to a modified method of the Sanger's method (Proc. Natl. Acad. Sci., USA, 74, p5463, 1977) using ^{T7}Sequencing™ Kit (Pharmacia). Namely, alkaline denaturated double-stranded DNA was used as a template, and primer-extension reaction using T7 DNA polymerase was nucleotide-specifically terminated by uptake of dideoxynucleotides, and after development by 8% urea - 6% polyacrylamide gel electrophoresis, incorporation of α[³²P]-dATP was detected by autoradiography.

T3 Primer 5′ -ATTAACCCTCACTAAAG-3′ (SEQ ID NO: 5)

T7 Primer 5′ -AATACGACTCACTATAG-3′ (SEQ ID NO: 6)

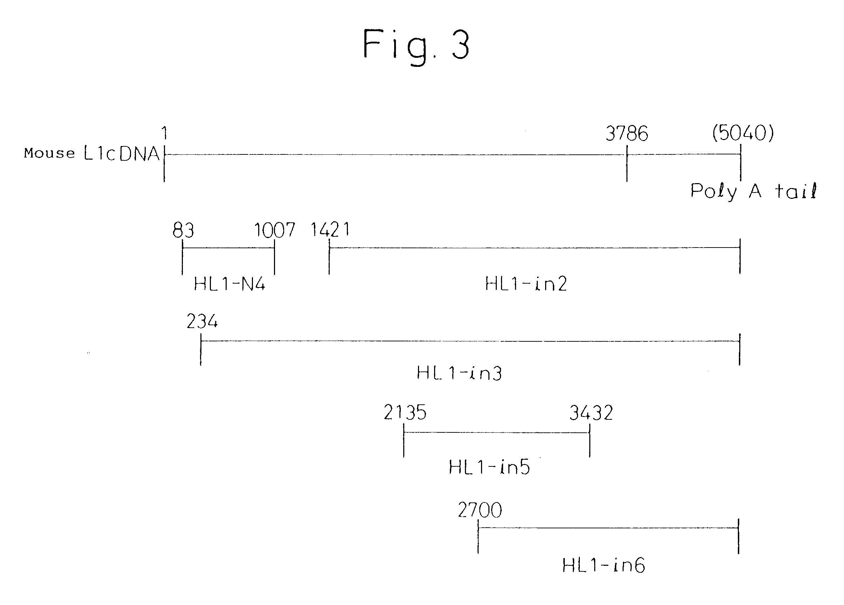

As a result, by comparison with mouse cDNA sequence already reported (Nature 334, p701 - 703, 1988), it was found that the HL-1-N4 corresponds to the 83 - 1007 position of mouse cDNA nucleotide sequence, HL1-in2 corresponds to the 1421-poly A tail, HL1-in3 corresponds to 234-poly A tail, HL1-in5 corresponds to 2135 - 3432, and HL1-in6 corresponds to the 2700-poly A tail (see, Fig. 3). Note, although homology (< 50%) was found between HL1-in1 and mouse L1 cDNA, and between HL1-in4 and mouse L1 cDNA, they were different from L1.

### Example 8

### On the basis of the restriction enzyme map shown in

Fig. 2 for HL1-in3, and the restriction enzyme map shown in Fig. 3 for HL1-N4, DNA was fragmented, and each fragment was inserted into a vector. Namely, various restriction enzyme-cleaved DNA fragments were subjected to an agarose gel electrophoresis, purified with a Geneclean (Bio 101), and inserted into a vector Bluescript SK- at a corresponding restriction enzyme site using T4 DNA ligase (Takara Shuzo). Sau 3AI-cleaved DNA fragment was inserted to BamHI site. The DNA thus obtained was used to transform E. coli XL1-blue or E. coli HB101 according to the above-mentioned method to obtain a large amount of DNA.

These DNAs were used as templates, and the above-mentioned primers T3, T7 and KS Primer ; 5' -CGAGGTCGACGGTATCG-3' (SEQ ID NO: 7) SK Primer ; 5' -TCTAGAACTAGTGGATC-3' (SEQ ID NO: 8) M13-20 Primer ; 5′ -GTAAAACGACGGCCAGT-3′ (SEQ ID NO: 9) Reverse Primer; 5'-CAGGAAACAGCTATGAC-3' (SEQ ID NO: 10), as well as the above-mentioned primers PL1, PL2, PL3, and 
PL5; 5′ -CAGGACCATATTTGTTAATGGCAGTGACCCGAAAGGTGTAGTG-3′
(SEQ ID NO: 11)
 were used to determine nucleotide sequences according to the method described in Example 7.

Considering a overlap between HL1-N4 and HL1-in3 as shown in Example 7, they were linked to form a nucleotide sequence of L1 gene cDNA and deduced amino acid sequence, as shown in SEQ ID NO: 12. The HL1-N4 corresponds to the nucleotide sequence 1 - 924 of this sequence, and the HL1-in3 correspcnds to the sequence 151 - 4770 of this sequence. Amino acid sequences of HL1-N4 and HL1-in3 were deduced by determining a stop codon by comparison with the nucleotide sequence of mouse L1 cDNA.

### Example 9 Construction of human leukocytederived genomic DNA library

A leukocyte fraction was obtained from human blood, and high molecular weight genomic DNA was extracted according to a method described in Maniatis et al., Molecular Cloning, p280, 1982). Next, the genomic DNA was partially digested with restriction enzyme EcoRI. On the other hand, bacteriophage λL47.1 DNA previously prepared according to Maniatis et al., Molecular Cloning, p41, 1982) was cleaved with EcoRI to prepare an arm DNA wherein an internal sequence had been eliminated. Next, according to a method described in Maniatis et al., Molecular cloning, p286, 1982, the EcoRI-partially cleaved genomic DNA and the arm DNA of phage λL47.1 were ligated, in-vitro packaging was carried out, and the phage was infected to E. coli K802 and amplified to obtain a library.

### Example 10 Screening of human leukocyte-derived genomic DNA library

The human leucocyte-derived genomic DNA library was screened by plaque hybridization method according to Denton and Davis method described in Example 4. Namely, E. coli K802 was infected with phage from the human leukocyte-derived genomic DNA library, phages were transferred from an agar medium having phage plaques thereon to a Biodyne membrane, and screened according to the method described in Example 4. For the screening, the PLN described in Example 2(3) was radio-labeled with α[³²P]-dATP according to a random primer method (Anal. Biochem. 132, p6, 1983) and used as a probe, and detected by autoradiography. This probe is a PCR product from mouse mRNA between PL3 and PL4, and contains a sequence corresponding to 5'-terminal nucleotide sequence 1 to 366 of mouse L1 cDNA. As a result of the screening, one clone positive relating to this probe was obtained.

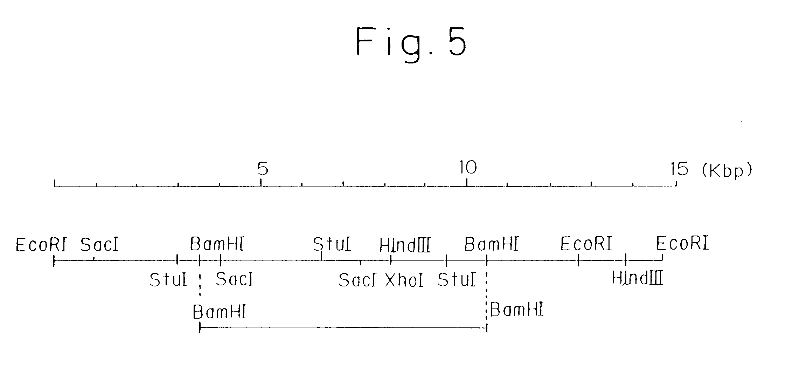

E. coli K802 was infected with phage from this clone, phage DNA was obtained according to a method described in Maniatis et al., Molecular Cloning, p80, and a restriction enzyme map of the inserted genomic DNA was prepared. This restriction enzyme map is shown in Fig. 5. Moreover, Southern blotting of the phage DNA was carried out using as a probe the PLN radio-labeled with α[³²P]-dATP, according to a method described in Maniatis et al., Molecular Cloning, p383, 1982. As a result, it was confirmed that a nucleotide sequence corresponding to 1 to 366 positions of mouse L1 cDNA was present on a BamHI fragment of about 7 Kbp.

Next, the fragment of about 7 Kbp liberated from the phage DNA by BamHI cleavage was separated by 0.7% agarose gel electrophoresis, and purified with a Geneclean Kit. This DNA fragment shown in Fig. 5 was inserted into a vector pBluescript SK-1 at BamHI cleavage site according to the same procedure as described in Example 2, the resulting vector was used to transform E. coli XL1-blue, which was then cultured, and a large amount of plasmid DNA was obtained by an alkaline denaturation method (Maniatis et al., Molecular Cloning, p368, 1982). This plasmid was designated as HL1-GN.

### Example 11 Analysis of genomic DNA clone HL1-GN

A partial nucleotide sequence of the plasmid HL1-GN obtained in Example 10 was determined according to the same procedure as described in Example 7.

Synthetic oligonucleotide PL4 corresponding to 1 to 24 positions of mouse L1 cDNA shown in Example 1, and a newly prepared oligonucleotide (designated as PL6) corresponding to a complementary chain of 63 to 80 position of the nucleotide sequence shown in SEQ ID NO: 12 were used as primers.

PL6:5' -AGGCTGATGTCATCTGTG-3'     (SEQ ID NO: 13)

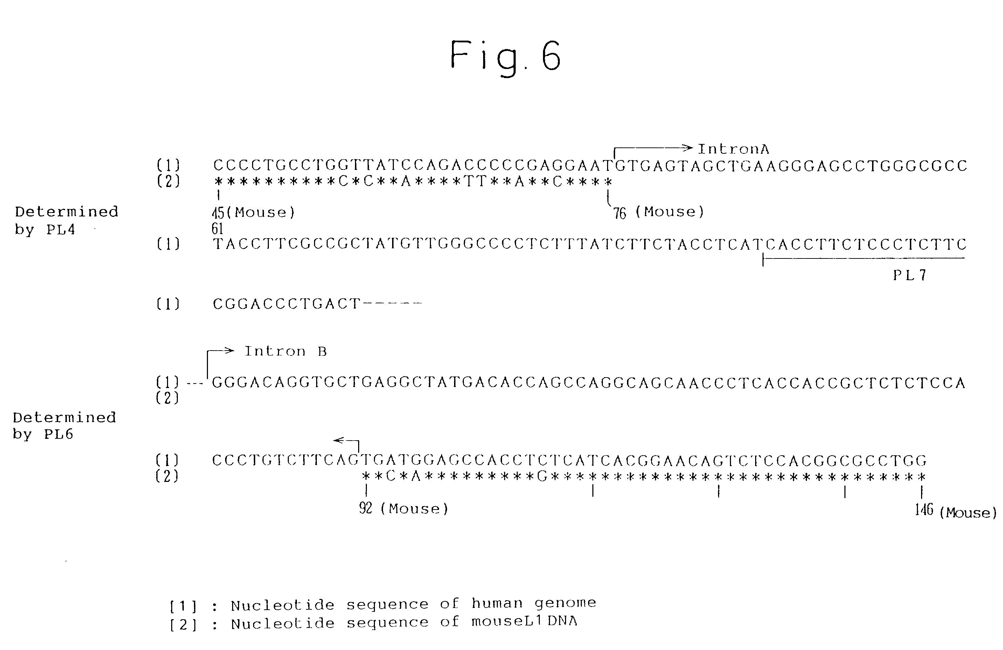

Sequences determined using PL4 and PL6 were shown in Fig. 6 comparing with a nucleotide sequence of mouse L1 cDNA. In both the sequence determined by PL4 and the sequence determined by PL6, there were sequences to be considered as introns between 76 - 77 positions and between 91 - 92 positions of the nucleotide sequence of mouse L1 cDNA respectively. They are designated as intron A and intron B.

Next, an oligonucleotide corresponding to a complementary sequence of a sequence (shown in Fig. 6) existing in the intron A was synthesized, and designated as PL7.

PL7:5' -CGGAAGAGGGAGAAGGTG-3' (SEQ ID NO: 14)

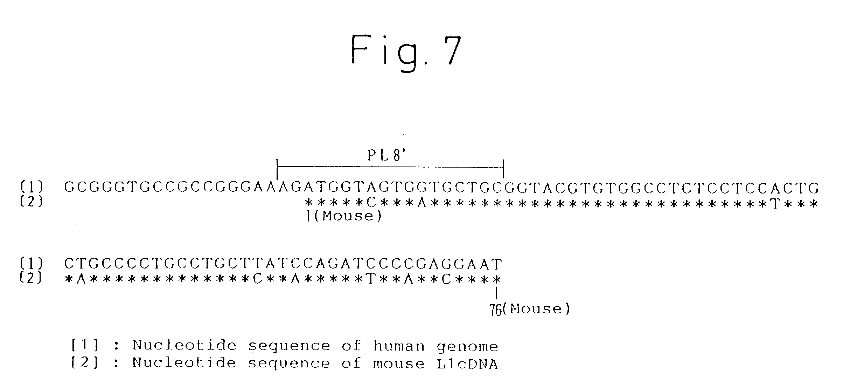

This PL7 was used to determine a partial nucleotide sequence of the plasmid HL1-GN. The sequence thus obtained was compared with the nucleotide sequence of mouse L1 cDNA to identify a sequence on human genome corresponding to the transcription start region of mouse L1 cDNA. A result is shown in Fig. 7.

### Example 12 Synthesis of cDNA from human neural tissue (?) -derived RNA by PCR method

An oligonucleotide wherein an XhoI recognizing site is linked to a human genomic sequence (PL8') corresponding to the mouse L1 cDNA start region was prepared as shown in Fig. 7, and was designated as PL8.

A complementary chain oligonucleotide of 127 to 148 positions of the nucleotide sequence shown in SEQ ID NO: 12 (corresponding to 221 to 242 positions of the nucleotide sequence of mouse L1 cDNA) was synthesized, and designated as PL9. The PL8 and PL9 have restriction enzyme sites XhoI and StyI respectively.

PL8:5′ -TTCTCGAGATGGTAGTGGTGCTGC-3′ (SEQ ID NO: 16)
Xho I recognition site

PL9:5′ -TCTTCCTTGGGTTTGAAGTGGA-3′   (SEQ ID NO: 17)
Sty I recognition site

On the other hand, according to the same procedure as described in Example 2(1), total RNA preparations were obtained from human adult brain, spinal cord cauda equina. 4 µg each of the RNA preparations were mixed, the mixture was extracted with phenol/chloroform (1:1) and with chloroform, sodium acetate was added thereon to a 0.3M final concentration, and ethanol precipitation was carried out. After that, cDNA was prepared using a reverse transcriptase by the same procedure as described in Example 2(2), and 40 polymerase chain reaction (PCR) temperature cycles of 94°C-2 minutes, 55°C-2.4 minutes and 72°C-3.5 minutes were carried out using the above-mentioned PL8 and PL9 as primers, and using Tag polymerase. The PCR product DNA was subjected to extraction with phenol and chloroform and ethanol precipitation, and the precipitate was dissolved in 50 µl of TE buffer. Next, 10 µl of this sample was cleaved with Xho I and Sty I (both from Takara Shuzo), and subjected to extraction with phenol and chloroform, and ethanol precipitation.

In addition, similarly, the plasmid HL1-N4 obtained in Example 5 was cleaved with Xho I and Sty I, a liberated fragment was removed by 0.7% agarose gel electrophoresis, and DNA of about 3.7 Kbp was purified using a Geneclean Kit. This DNA is a DNA fragment comprising a DNA corresponding to 140 to 924 positions of nucleotide sequence described in SEQ ID NO: 12 having a Sty I cleavage site at its 5'-end and linked to EcoRI cleavage site of vector pBluescript SK-.

Next, 25 ng of the restriction enzyme-cleaved PCR product and 100 ng of the DNA fragment obtained by cleaving plasmid HL1-N4 with the restriction enzyme were ligated with T4 DNA ligase according to the same procedure as described in Example 2(2). The reaction product was used to transform competent cells of E. coli HB101 to obtain transformants. Among them, 5 transformants were selected and cultured, a large amount of plasmid was prepared by an alkaline denaturation method, and nucleotide sequence was determined using T3 and T17 primers according to the same procedure as described in Example 7.

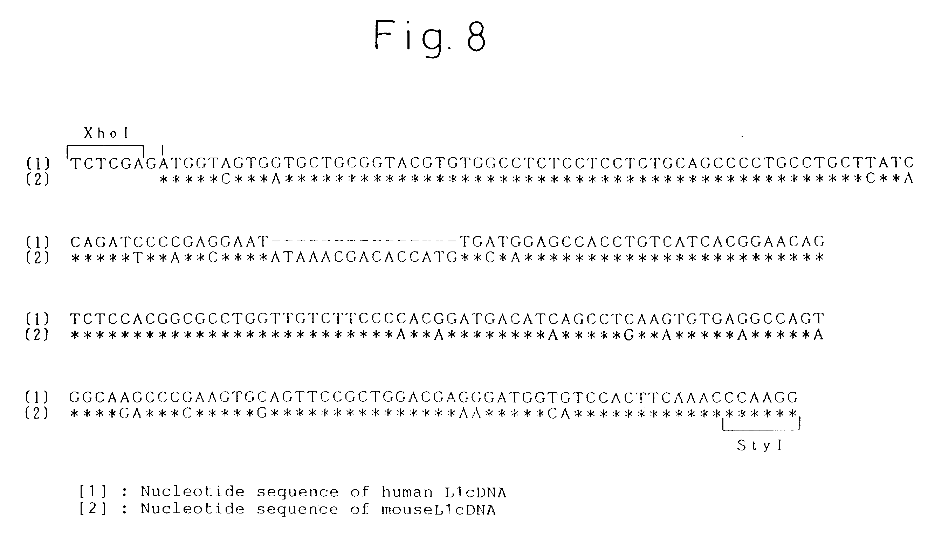

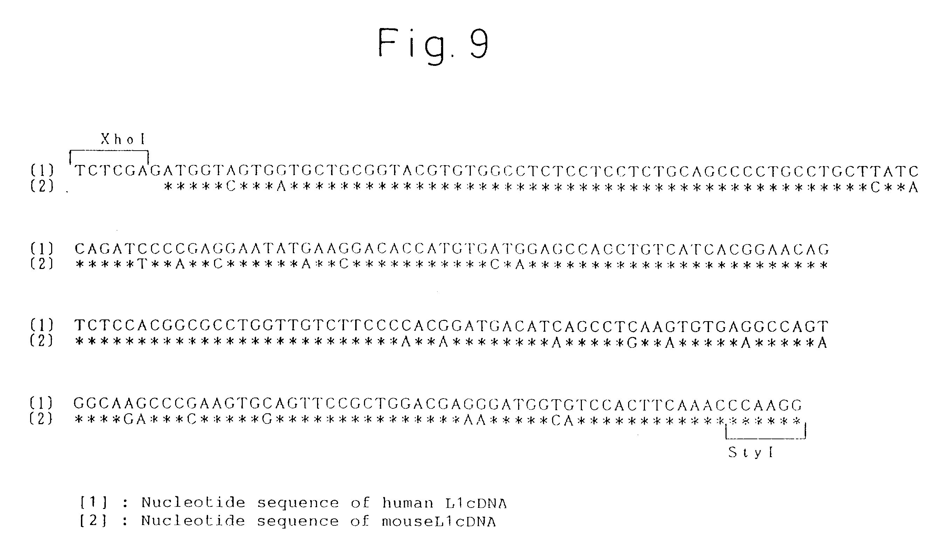

It was found that among the transformants, one clone contained a PCR product of about 230 bp and another clone contained a PCR product of about 250 bp, each inserted into plasmid HL1-N4 at its Xho I/Sty I cleavage sites. Among the plasmids derived from these clones, the former is designated as HL1-NPCR3 and the latter is designated as HL1-NPCR5, and sequences between Xho I and Sty I are shown in Figs. 8 and 9 respectively, comprising with the nucleotide sequence of mouse cDNA.

Among them Escherichia coli HB101 [HL1-NPCR3] containing the former plasmid was deposited with Fermentation Research Institute Agency of Industrial Science and Technology as FERM BP-3377 on April 30, 1991; and Escherichia coli HB101 [HL1-NPCR5] containing the latter plasmid was deposited as FERM BP-3401 on May 16, 1991.

A nucleotide sequence prepared by linking the HL1-NPCR3 and HL1-in3 described in Example 7 considering their overlapping portions, and an amino acid sequence predicted from the nucleotide sequence are described in SEQ ID NO: 17; and similarly, sequences prepared by linking HL1-NPCR5 and HL1-in3 are shown in SEQ ID NO: 18. The HL1-NPCR3 corresponds to 1 to 993 positions of SEQ ID NO: 17, and a sequence coding for a protein in HL1-in3 correspond 220 to 3759 positions of the same SEQ ID NO. The HL1-NPCR5 correspond 1 to 1008 positions of SEQ ID NO: 18, and a sequence coding for a protein is HL1-in3 corresponds to 235 to 3774 positions of the same SEQ ID NO.

### Example 13 Construction of plasmid containing human L1 entire cDNA

From HL1-in3, a DNA having a sequence 221 - 951 positions shown in SEQ ID NO: 17 was removed using Xho I and Sph I, and a remaining DNA fragment was purified using a Geneclean Kit according to a conventional procedure. Similarly, fragments liberated from HL1-NPCR3 and HL1-NPCR5 using Xho I and Sph I (the former has a nucleotide sequence 1 to 951 shown in SEQ ID NO: 13, and the latter has a nucleotide sequence 1 to 966 shown in SEQ ID NO: 18) were isolated. Next, these DNA were ligated with a DNA fragment derived from the above-mentioned HL1-in3 using T4 DNA ligase respectively.

As a result, two plasmids were obtained wherein DNAs containing a nucleotide sequence coding for proteins shown in SEQ ID NOs: 17 and 18 had been inserted into a vector pBluescript SK- at Xho I-EcoRI cleavage sites respectively. The former and the latter were tentatively designated as R3 and R5 respectively. In these plasmids, a non-protein-coding region from the BamHI cleavage site downstream of the stop codon TAG to the BamHI cleavage site present in the vector was deleted and the remaining DNA was ligated.

As a result, two plasmids were obtained wherein DNAs containing nucleotide sequences shown in SEQ ID NOs: 17 and 18 had been inserted into the vector pBluescrpit SK-1 at XhoI-BamHI cleavage sites. Both of these plasmids contained a sequence from the start codon ATG to the stop codon TAG. Among these plasmids, the former is designated as BSSK-HL1 and the latter is designated as BSSK-HL1(+).

### Example 14 Expression of HL1 and HL1(+) in COS cells

The plasmid BSSK-HL1(+) was used to transform competent cells of E. coli HB101, and the transformant was cultured to obtain a large amount of plasmid. From this plasmid, cDNA (HL1(+)) containing an entire coding region for human L1 was liberated using XhoI and BamHI, and the cDNA was purified using a Geneclean Kit. This HL1(+) was inserted into an expression vector pSVL (Pharmacia) at polylinker site (between XhoI - BamHI) to construct an expression vector for a human L1. This vector is designated as pSVLHL1(+).

In exactly the same manner, plasmid BSSK-HL1 was used to construct an expression vector pSVLHL1.

For introduction of pSVLHL1(+) and pSVLHL1, COS cells were used. The COS cells were cultured Dulbecco's Modified Eagle Medium (DMEM) containing 10% bovine fetal serum in 5% CO₂. On the day before introduction of gene, the cells were subcultured at a concentration of 5 × 10⁵ cells/6 cm culture dish. 2 µg of pSVHL1(+) DNA was introduced into the cells by DEAE-Dextran method (see, Sambrook et al., Molecular Cloning, a Laboratory Manual, Second edition, 1989, Chapter 16, 45 page). After the introduction of gene, the cells were cultured for 48-72 hours continuously, and recovered. The cells were washed with TBS (10 mM Tris-HCl, pH 7.4, 0.9% NaCl), and ten times disrupted with a teflon homogenizer in buffer A (10 mM Tris-HCl, pH 8.0, 1 mM EDTA, 1 mM phenylmethyl sulfonyl fluoride (PMSF), 0.01 mM pepstatine A, 0.2 V/ml aprotinin).

The homogenate was centrifuged at 3,300 xg for 3 minutes to remove a nucleus fraction, and the supernatant was further centrifuged at 100,000 xg for 60 minutes to obtain a crude membrane fraction. From this membrane fraction, membrane protein was extracted with buffer A containing 0.1% deoxycholic acid and designated as extract A. The same procedure was applied to pSVLHL1 to obtain extract B. As a control experimental group, a membrane protein was extracted from COS cells into which gene had not been introduced to obtain extract C.

### Example 15 Confirmation of human L1 protein expressed in COS cells

The extracts A, B and C described in Example 14 were separately developed by 8% polyacrylamide gel electrophoresis, to transfer protein onto Biodyn (?) nylon membrane. After that, human L1 protein was detected by an antigen-antibody reaction. The antibody used was obtained by gene engineering a nucleotide sequence (corresponding to 3460 - 3780 positions of mouse L1 cDNA) coding for an intracellular region from rat L1 cDNA, cloned from a rat brain cDNA library, inserting the sequence into an expression vector pMAL (New England Bio Labs) at a cloning site present therein, and immunizing a rabbit with a protein expressed in E. coli with Freund complete adjuvant (DIFCO).

Predicted amino acid sequence of this expressed protein conforms to a predicted amino acid sequence corresponding to 3436 to 3759 positions (SEQ ID NO: 17) of human L1 cDNA nucleotide sequence. By using the anti-rat L1 antibody recognizing an amino acid sequence common between human and rat, expression of human L1 protein in the extracts A and B was confirmed.

### Example 16 Construction of CHO cell expression vector pDE

Expression vector pDE was constructed from the following 3 DNA fragments.

CDM8 (B. Seed, Nature, 329, 840 - 842, 1987) was sequentially treated with BanI, T4 DNA polymerase and AsuII to obtain a DNA fragment containing a cytomegalovirus (CMV) enhancer region of about 660 bp. This DNA fragment was ligated with a DNA fragment containing an EFIx promoter region obtained by sequentially treating pEF-BOS (S. Mizushima and S. Nagata, Nucleic Acid Res. 18, 5322, 1990) with HindIII, T4 DNA polymerase and XbaI. In addition, both of the ends were blunt-ended by treating with T4 DNA polymerase.

A EcoRI-BamI DNA fragment containing adenovirus type 2 major late promoter and dihydroforate reductase (DHFR) gene from pAd26SVpA (R. J. Kaufman et al., Mol. Cell, Biol, 2, 1304, 1982) was inserted into pdKCR (R. Fukunaga et al., Proc, Natl. Acod, USA, 81, 5086, 1984) at EcoRI cleavage site. The resulting plasmid is designated as pdR. The pdKCR was treated with FokI and Klenow fragment, and linked to BamHI linker (Takara Shuzo), and cleaved with BamHI and PvuII to obtain a DNA fragment of about 180 bp containing SV40 late promoter lacking an enhancer region. This DNA fragment was inserted into pdR at BamHI, PvuII cleavage sites to obtain plasmid pDRΔ SVE containing a DHFR gene downstream of the SV40 late promoter. This plasmid was sequentially treated with BamHI, Mung Bean Nuclease and PvuI to obtain a DNA fragment containing an SV40 late promoter, a DHFR gene, SV40 poly A region and a part of an ampicillin resistant gene.

A linker comprising 2 oligonucleotides (SEQ ID NOs: 19 and 20) synthesized using a DNA synthesizer (Applied Biosystems) was inserted into pSP73 (Promega) at Aat II, Nsp 7524I cleavage sites. To Bgl II cleavage site of the resulting plasmid pSP73Δ, a BamHI DNA fragment containing a neomycin resistance gene and SV40 poly A region obtained from pMAMneo (Clontech) was inserted. A resulting plasmid pSP73 neopA was treated with HpaI and PvuI to obtain a DNA fragment containing an SV40 poly A region, an origin of replication for E. coli and a part of an ampicillin resistance gene.

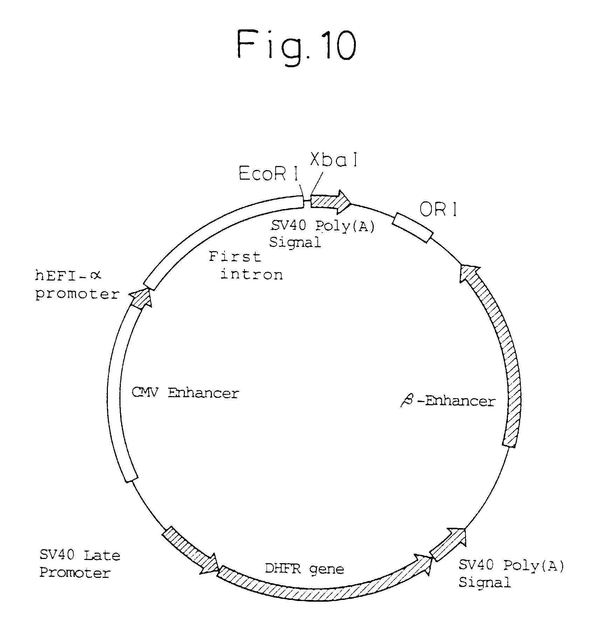

These 3 DNA fragments, i.e., a DNA fragment containing EF1α promoter, a DNA fragment containing a DHFR gene and a DNA fragment containing an origin of replication were ligated to obtain an expression vector. This is designated as pDE (Fig. 10).

### Example 17 Expression of an full length human L1 gene in CHO cells

Two oligonucleotides shown in SEQ ID NOs: 21 and 22 containing EcoRI site and a part of L1cDNA5' end were synthesized by a DNA synthesizer and used as a linker. The plasmid R5 in Example 13 was cleaved with Af1III to obtain a DNA fragment of about 2.3 kb of L1cDNA (21-2330 in SEQ ID NO: 18). This DNA fragment was ligated with the synthetic linker, and treated with EcoRI and SmaI to obtain a DNA fragment of about 2.1 kb containing 5'- side of L1 gene. Next, a SmaI-BamHI DNA fragment of about 1.7 kb containing 3'- side of L1cDNA was obtained. These 2 DNA fragments were inserted into pSP73 at EcoRI, BamHI cleavage sites. A resulting plasmid containing a L1 gene modified at the 5'- terminus and lacking a major part of 3'-nontranslation region is designated as pXXKOZ. A EcoRI-XbaI DNA fragment containing L1 gene in XXKOZ was inserted into the EcoRI, XhaI cleavage site of pDE described in Example 16 to construct an entire length L1 expression vector pDELB.

The pDELB was cleaved with Pvu I and introduced into CHO cell DXB11 (R. J. Kaufman, Methods in Enzymology, 185, 537 - 566, 1990) using lipofectin, and the cells were cultured according to Kaufman's method (R. J. Kaufman, Methods In Enzymology, 185, 537 - 566, 1990) using α-MEM (GIBCO) and treated with methotrexate (MTX) for gene amplification to obtain 20 nM MTX resistant strains. In the same manner as in Example 15, expression of L1 was confirmed by disrupting the cells, SDS-PAGE, and Western blotting. The primary antibody is that to intracellular region of rat L1, and JI strain and JE strain exhibiting strong L1-specific band around 200K and 80K were obtained.

### Example 18 Expression of secreted human L1 in CHO cells

Using a DNA synthesizer a primer L1F (SEQ ID NO: 23) containing a part of L1cDNA (3214 - 3240 positions in SEQ ID NO: 18) and a primer L1R (SEQ ID NO: 24) containing a part of L1cDNA sequence (3337 - 3357 positions in SEQ ID NO: 18) and a stop codon were synthesized. A DNA fragment of about 150 bp was obtained by PCR (94°C - 1.5 minutes, 55°C - 1.5 minutes, 72°C - 1.5 minutes) using pXXKOZ as a template, two primers and a GeneAmp PCR Reagent Kit (Perkin-Elmer Cetus Instruments). An EcoRI-XbaI fragment containing L1 gene of pXXKOZ was inserted into pUC19 at EcoRI, Xba cleavage sites to obtain plasmid pL1Bint, which was then treated with SacI and XbaI to eliminate the 3'- end of L1 gene, and a DNA fragment formed by treatment of the PCR product with SacI and XbaI was inserted therein.

An EcoRI-XbaI fragment of the resulting plasmid pL1Bsol containing an L1 gene was inserted into EcoRI, XbaI sites of pDE described in Example 16. A resulting plasmid is designated as pDELBsol. In a similar manner as in Example 17, pDELBsol was introduced into CHO cell DXB11 strain using lipofectin to obtain a 20 nM MTX resistant cell line. A culture medium obtained by using serum-free medium ASF104 (Ajinomoto) was concentrated by a centricon-100, and Western blotting was carried out using SDS-PAGE and anti-L1 antibody Ig. To prepare anti-L1 antibody Ig, in a similar manner as in Example 15, an XhoI-HindIII DNA fragment (containing 1-2385 positions in SEQ ID NO: 17) of L1 cDNA prepared from plasmid R3 described in Example 13 was inserted into pMAL at HindIII and SalI. A resulting plasmid was expressed in E. coli, and the expression product and Freund complete adjuvant (DIFCO) were used to immunize a rabbit to obtain anti-serum. A result of Western blotting confirmed a human L1 specific band of about 200K.

### Example 19 Effects of full length human L1 molecule on the neurite extension of cultured cerebellum neuron

The experimental method was slightly modified from the procedure of Schnitzer J and Schachner M (J. Neuroimmunology, 1: 429 - 456, 1981).

The cerebellum was isolated, after soaking mice in 70% ethanol from animals of 0 to 4 days old (ICR, Charles River, Japan) by decapitating. Meninges and choroid plexus were recovered by tweezers from the cerebellum under a microscope, while bathing the tissue ice cold (4°C) Hanks BSS (calcium magnesium free) (CMF-HBSS) (Sigma) solution containing 10 mM HEPES and 0.1 mM CaCl₂, whose pH had been adjusted to 7.4 with 1N NaOH. The cerebellum thus obtained was cut into to 3 pieces, and allowed to stand in CMF-HBSS solution containing 0.05% DNase and 0.2 mg Dispase (Boehringer Mannheim, Germany) at a room temperature for 7 minutes. Next, the supernatant solution was discarded by decautation and to the tissue, 0.05% DNase/CMF-HBSS solution was added (1 ml/3 pieces of one cerebellum). The tissue was gently homogenized by pipetting 15 times in a 15 ml centrifuge tube using a Pasteur pipette whose tip has been made round by a gas burner.

The suspension thus prepared was allowed to stand for 5 minutes on ice. Dispered cells were recovered as supernatant from the top 4/5 portion of the supernatant. The single cell suspension thus obtained was centrifuged at 4°C, 60 xg for 5 minutes, and the pellet was resuspended in a 3 ml of 0.05% DNase/CMF-HBSS solution. After repeating this procedure, the cell pellet was resuspended in a culture medium (Eagle's salt-containing Eagle's basal medium +L- glutamine (final concentration 292 mg/L), glucose (final concentration 5g/L), NaHCO₃ (final concentration 2200 mg/L), kanamycin (final concentration 50 mg/L), 8% bovine fetal serum (Flow Lab.), and 2% calf serum (Flouw Lab.)) and the number of viable cells was adjusted to 1 × 10⁵ as by counting using a hemacytometer with trypan blue staining.

JE cell line and JI cell line of Example 17 were separately plated on cover glasses (diameter 13 mm, thickness 0.12 to 0.17 mm) to near confluent. Three hours later, 100 µl of the above-prepared suspension of cerebellar neuron was overelaied on JE or JI cells, which were then cultured at 37°C in 5% CO₂, and 95% air atmosphere in the humidified chamber. After 24 hours, neutrite-extension was compared with that on CHO cells to which L1 gene had not been transfected. As a result, only cerebellar neurons cultured on L1 expressing cell lines JE and JI demonstrated remarkable neutrite-extension.

### Example 20 Effect of the secreted form of human L1 molecule on the neutrite-extension of cerebellar neuron

A concentrate (5 µl) of the culture supernatant prepared in Example 18 was dropped on a cover glass coated with polylysin (ICN Immuno Biol.) (the poly-L-lysine coated cover glass was prepared by soaking a cover glass in a solution of 100 µg/ml polylysin, at 37°C for 3 hours in a CO₂ incubator, aspirating the solution, and washing with distilled water), and the cover glass was allowed to stand at 37°C for 3 hours in a CO₂ incubator and once washed with PBS(-) to complete the coating process. The cerebellar neurons prepared in Example 19 were cultured on the cover glass coated with the culture supernatant concentrate, at 37°C in a 5% CO₂, 95% air atmosphere to test neurite-extension. Neurite-extension was evident only on the neurous cultured on the cover glass coated with the culture supernatant concentrate containing secretory type L1.

### [Industrial Applicability]

The present human neural cell adhesion molecule or derivatives thereof was promised as medicaments for treating diseases of nervous system.

Reference to deposited microorganisms under Rule 13-2, and depository authority
Ministry of International Trade and Industry, Fermentation Research Institute Agency of Industrial Science and Technology,
Address: 1-3 Higashi 1-chome, Ibaraki-ken, 305 Japan Number and data of deposition:
1. Escherichia coli HB101 [HL1-N4]
   Deposition No: FERM BP-3257
   Deposition Date: January 30, 1991
2. Escherichia coli HB101 [HL1-in3]
   Deposition No: FERM BP-3256
   Deposition Date: January 30, 1991
3. Escherichia coli HB101 [HL1-NPCR3]
   Deposition No: FERM BP-3377
   Deposition Date: April 30, 1991
4. Escherichia coli HB101 [HL1-NPCR5]
   Deposition No: FERM BP-3401
   Deposition Date: May 16, 1991

### [SEQUENCE LISTING]

### SEQ ID NO: 1

SEQUENCE LENGTH: 28
SEQUENCE TYPE: Nucleic acid
STRANDNESS: Single
TOPOLOGY: Linear
MOLECULE TYPE: Synthetic DNA
SEQUENCE:

CCGGATCCTA TTCTAGGGCT ACTGCAGG 28

### SEQ ID NO: 2

SEQUENCE LENGTH: 35
SEQUENCE TYPE: Nucleic acid
STRANDNESS: Single
TOPOLOGY: Linear
MOLECULE TYPE: Synthetic DNA
SEQUENCE:

GAAGGCCTTT GGCAGCAGCC AGCCATCTCT CAACG 35

### SEQ ID NO: 3

SEQUENCE LENGTH: 42
SEQUENCE TYPE: Nucleic acid
STRANDNESS: Single
TOPOLOGY: Linear
MOLECULE TYPE: Synthetic DNA
SEQUENCE:

GGCAGTTCCT AGCTTATTGC TGGCATAGCA GCGATAGATG CC 42

### SEQ ID NO: 4

SEQUENCE LENGTH: 21
SEQUENCE TYPE: Nucleic acid
STRANDNESS: Single
TOPOLOGY: Linear
MOLECULE TYPE: Synthetic DNA
SEQUENCE:

ATGGTCGTGA TGCTGCGGTA C 21

### SEQ ID NO: 5

SEQUENCE LENGTH: 17
SEQUENCE TYPE: Nucleic acid
STRANDNESS: Single
TOPOLOGY: Linear
MOLECULE TYPE: Synthetic DNA
SEQUENCE:

ATTAACCCTC ACTAAAG 17

### SEQ ID NO: 6

SEQUENCE LENGTH: 17
SEQUENCE TYPE: Nucleic acid
STRANDNESS: Single
TOPOLOGY: Linear
MOLECULE TYPE: Synthetic DNA
SEQUENCE:

AATACGACTC ACTATAG 17

### SEQ ID NO: 7

SEQUENCE LENGTH: 17
SEQUENCE TYPE: Nucleic acid
STRANDNESS: Single
TOPOLOGY: Linear
MOLECULE TYPE: Synthetic DNA
SEQUENCE:

CGAGGTCGAC GGTATCG 17

### SEQ ID NO: 8

SEQUENCE LENGTH: 17
SEQUENCE TYPE: Nucleic acid
STRANDNESS: Single
TOPOLOGY: Linear
MOLECULE TYPE: Synthetic DNA
SEQUENCE:

TCTAGAACTA GTGGATC 17

### SEQ ID NO: 9

SEQUENCE LENGTH: 17
SEQUENCE TYPE: Nucleic acid
STRANDNESS: Single
TOPOLOGY: Linear
MOLECULE TYPE: Synthetic DNA
SEQUENCE:

GTAAAACGAC GGCCAGT 17

### SEQ ID NO: 10

SEQUENCE LENGTH: 17
SEQUENCE TYPE: Nucleic acid
STRANDNESS: Single
TOPOLOGY: Linear
MOLECULE TYPE: Synthetic DNA
SEQUENCE:

CAGGAAACAG CTATGAC 17

### SEQ ID NO: 11

SEQUENCE LENGTH: 43
SEQUENCE TYPE: Nucleic acid
STRANDNESS: Single
TOPOLOGY: Linear
MOLECULE TYPE: Synthetic DNA
SEQUENCE:

CAGGACCATA TTTGTTAATG GCAGTGACCC GAAAGGTGTA GTG 43

### SEQ ID NO: 12

SEQUENCE LENGTH: 3690
SEQUENCE TYPE: Nucleic acid
STRANDNESS: Double
TOPOLOGY: Linear
MOLECULE TYPE: cDNA
SEQUENCE:

CAC CAT GTG ATG GAG CCA CCT GTC ATC ACG GAA CAG TCT CCA CGG CGC 48
His His Val Met Glu Pro Pro Val Ile Thr Glu Gln Ser Pro Arg Arg
1               5                   10                  15
CTG GTT GTC TTC CCC ACA GAT GAC ATC AGC CTC AAG TGT GAG GCC AGT 96
Leu Val Val Phe Pro Thr Asp Asp Ile Ser Leu Lys Cys Glu Ala Ser
            20                  25                  30

GGC AAG CCC GAA GTG CAG TTC CGC TGG ACG AGG GAT GGT GTC CAC TTC 144
Gly Lys Pro Glu Val Gln Phe Arg Trp Thr Arg Asp Gly Val His Phe
        35                  40                  45
AAA CCC AAG GAA GAG CTG GGT GTG ACC GTG TAC CAG TCG CCC CAC TCT 192
Lys Pro Lys Glu Glu Leu Gly Val Thr Val Tyr Gln Ser Pro His Ser
    50                  55                  60
GGC TCC TTC ACC ATC ACG GGC AAC AAC AGC AAC TTT GCT CAG AGG TTC 240
Gly Ser Phe Thr Ile Thr Gly Asn Asn Ser Asn Phe Ala Gln Arg Phe
65                  70                  75                  80
CAG GGC ATC TAC CGC TGC TTT GCC AGC AAT AAG CTG GGC ACC GCC ATG 288
Gln Gly Ile Tyr Arg Cys Phe Ala Ser Asn Lys Leu Gly Thr Ala Met
                85                  90                  95
TCC CAT GAG ATC CGG CTC ATG GCC GAG GGT GCC CCC AAG TGG CCA AAG 336
Ser His Glu Ile Arg Leu Met Ala Glu Gly Ala Pro Lys Trp Pro Lys
            100                 105                 110
GAG ACA GTG AAG CCC GTG GAG GTG GAG GAA GGG GAG TCA GTG GTT CTG 384
Glu Thr Val Lys Pro Val Glu Val Glu Glu Gly Glu Ser Val Val Leu
        115                 120                 125
CCT TGC AAC CCT CCC CCA AGT GCA GAG CCT CTC CGG ATC TAC TGG ATG 432
Pro Cys Asn Pro Pro Pro Ser Ala Glu Pro Leu Arg Ile Tyr Trp Met
    130                 135                 140
AAC AGC AAG ATC TTG CAC ATC AAG CAG GAC GAG CGG GTG ACG ATG GGC 480
Asn Ser Lys Ile Leu His Ile Lys Gln Asp Glu Arg Val Thr Met Gly
145                 150                 155                 160

CAG AAC GGC AAC CTC TAC TTT GCC AAT GTG CTC ACC TCC GAC AAC CAC 528
Gln Asn Gly Asn Leu Tyr Phe Ala Asn Val Leu Thr Ser Asp Asn His
                165                 170                 175
TCA GAC TAC ATC TGC CAC GCC CAC TTC CCA GGC ACC AGG ACC ATC ATT 576
Ser Asp Tyr Ile Cys His Ala His Phe Pro Gly Thr Arg Thr Ile Ile
            180                 185                 190
CAG AAG GAA CCC ATT GAC CTC CGG GTC AAG GCC ACC AAC AGC ATG ATT 624
Gln Lys Glu Pro Ile Asp Leu Arg Val Lys Ala Thr Asn Ser Met Ile
        195                 200                 205
GAC AGG AAG CCG CGC CTG CTC TTC CCC ACC AAC TCC AGC AGC CAC CTG 672
Asp Arg Lys Pro Arg Leu Leu Phe Pro Thr Asn Ser Ser Ser His Leu
    210                 215                 220
GTG GCC TTG CAG GGG CAG CCA TTG GTC CTG GAG TGC ATC GCC GAG GGC 720
Val Ala Leu Gln Gly Gln Pro Leu Val Leu Glu Cys Ile Ala Glu Gly
225                 230                 235                 240
TTT CCC ACG CCC ACC ATC AAA TGG CTG CGC CCC AGT GGC CCC ATG CCA 768
Phe Pro Thr Pro Thr Ile Lys Trp Leu Arg Pro Ser Gly Pro Met Pro
                245                 250                 255
GCC GAC CGT GTC ACC TAC CAG AAC CAC AAC AAG ACC CTG CAG CTG CTG 816
Ala Asp Arg Val Thr Tyr Gln Asn His Asn Lys Thr Leu Gln Leu Leu
            260                 265                 270
AAA GTG GGC GAG GAG GAT GAT GGC GAG TAC CGC TGC CTG GCC GAG AAC 864
Lys Val Gly Glu Glu Asp Asp Gly Glu Tyr Arg Cys Leu Ala Glu Asn
        275                 280                 285

TCA CTG GGC AGT GCC CGG CAT GCG TAC TAT GTC ACC GTG GAG GCT GCC 912
Ser Leu Gly Ser Ala Arg His Ala Tyr Tyr Val Thr Val Glu Ala Ala
    290                 295                 300
CCG TAC TGG CTG CAC AAG CCC CAG AGC CAT CTA TAT GGG CCA GGA GAG 960
Pro Tyr Trp Leu His Lys Pro Gln Ser His Leu Tyr Gly Pro Gly Glu
305                 310                 315                 320
ACT GCC CGC CTG GAC TGC CAA GTC CAG GGC AGG CCC CAA CCA GAG GTC 1008
Thr Ala Arg Leu Asp Cys Gln Val Gln Gly Arg Pro Gln Pro Glu Val
                325                 330                 335
ACC TGG AGA ATC AAC GGG ATC CCT GTG GAG GAG CTG GCC AAA GAC CAG 1056
Thr Trp Arg Ile Asn Gly Ile Pro Val Glu Glu Leu Ala Lys Asp Gln
            340                 345                 350
AAG TAC CGG ATT CAG CTG GGC GCC CTG ATC CTG AGC AAC GTG CAG CCC 1104
Lys Tyr Arg Ile Gln Leu Gly Ala Leu Ile Leu Ser Asn Val Gln Pro
        355                 360                 365
AGT GAC ACA ATG GTG ACC CAA TGT GAG GCC CGC AAC CGG CAC GGG CTC 1152
Ser Asp Thr Met Val Thr Gln Cys Glu Ala Arg Asn Arg His Gly Leu
    370                 375                 380
TTG CTG GCC AAT GCC TAC ATC TAC GTT GTC CAG CTG CCA GCC AAG ATC 1200
Leu Leu Ala Asn Ala Tyr Ile Tyr Val Val Gln Leu Pro Ala Lys Ile
385                 390                 395                 400
CTG ACT GCG GAC AAT CAG ACG TAC ATG GCT GTC CAG GGC AGC ACT GCC 1248
Leu Thr Ala Asp Asn Gln Thr Tyr Met Ala Val Gln Gly Ser Thr Ala
                405                 410                 415

TAC CTT CTG TGC AAG GCC TTC GGA GCG CCT GTG CCC AGT GTT CAG TGG 1296
Tyr Leu Leu Cys Lys Ala Phe Gly Ala Pro Val Pro Ser Val Gln Trp
            420                 425                 430
CTG GAC GAG GAT GGG ACA ACA GTG CTT CAG GAC GAA CGC TTC TTC CCC 1344
Leu Asp Glu Asp Gly Thr Thr Val Leu Gln Asp Glu Arg Phe Phe Pro
        435                 440                 445
TAT GCC AAT GGG ACC CTG GGC ATT CGA GAC CTC CAG GCC AAT GAC ACC 1392
Tyr Ala Asn Gly Thr Leu Gly Ile Arg Asp Leu Gln Ala Asn Asp Thr
    450                 455                 460
GGA CGC TAC TTC TGC CTG GCT GCC AAT GAC CAA AAC AAT GTT ACC ATC 1440
Gly Arg Tyr Phe Cys Leu Ala Ala Asn Asp Gln Asn Asn Val Thr Ile
465                 470                 475                 480
ATG GCT AAC CTG AAG GTT AAA GAT GCA ACT CAG ATC ACT CAG GGG CCC 1488
Met Ala Asn Leu Lys Val Lys Asp Ala Thr Gln Ile Thr Gln Gly Pro
                485                 490                 495
CGC AGC ACA ATC GAG AAG AAA GGT TCC AGG GTG ACC TTC ACG TGC CAG 1536
Arg Ser Thr Ile Glu Lys Lys Gly Ser Arg Val Thr Phe Thr Cys Gln
            500                 505                 510
GCC TCC TTT GAC CCC TCC TTG CAG CCC AGC ATC ACC TGG CGT GGG GAC 1584
Ala Ser Phe Asp Pro Ser Leu Gln Pro Ser Ile Thr Trp Arg Gly Asp
        515                 520                 525
GGT CGA GAC CTC CAG GAG CTT GGG GAC AGT GAC AAG TAC TTC ATA GAG 1632
Gly Arg Asp Leu Gln Glu Leu Gly Asp Ser Asp Lys Tyr Phe Ile Glu
    530                 535                 540

GAT GGG CGC CTG GTC ATC CAC AGC CTG GAC TAC AGC GAC CAG GGC AAC 1680
Asp Gly Arg Leu Val Ile His Ser Leu Asp Tyr Ser Asp Gln Gly Asn
545                 550                 555                 560
TAC AGC TGC GTG GCC AGT ACC GAA CTG GAT GTG GTG GAG AGT AGG GCA 1728
Tyr Ser Cys Val Ala Ser Thr Glu Leu Asp Val Val Glu Ser Arg Ala
                565                 570                 575
CAG CTC TTG GTG GTG GGG AGC CCT GGG CCG GTG CCA CGG CTG GTG CTG 1776
Gln Leu Leu Val Val Gly Ser Pro Gly Pro Val Pro Arg Leu Val Leu
            580                 585                 590
TCC GAC CTG CAC CTC GTG ACG CAG AGC CAG GTG CGC GTG TCC TGG AGT 1824
Ser Asp Leu His Leu Val Thr Gln Ser Gln Val Arg Val Ser Trp Ser
        595                 600                 605
CCT GCA GAA GAC CAC AAT GCC CCC ATT GAG AAA TAT GAC ATT GAA TTT 1872
Pro Ala Glu Asp His Asn Ala Pro Ile Glu Lys Tyr Asp Ile Glu Phe
    610                 615                 620
GAG GAC AAG GAA ATG GCG CCT GAA AAA TGG TAC AGT CTG GGC AAG GTT 1920
Glu Asp Lys Glu Met Ala Pro Glu Lys Trp Tyr Ser Leu Gly Lys Val
625                 630                 635                 640
CCA GGG AAC CAG ACC TCT ACC ACC CTC AAG CTG TCG CCC TAT GTC CAC 1968
Pro Gly Asn Gln Thr Ser Thr Thr Leu Lys Leu Ser Pro Tyr Val His
                645                 650                 655
TAC ACC TTT AGG GTT ACT GCC ATA AAC AAA TAT GGC CCC GGG GAG CCC 2016
Tyr Thr Phe Arg Val Thr Ala Ile Asn Lys Tyr Gly Pro Gly Glu Pro
            660                 665                 670

AGC CCG GTC TCT GAG ACT GTG GTC ACA CCT GAG GCA GCC CCA GAG AAG 2064
Ser Pro Val Ser Glu Thr Val Val Thr Pro Glu Ala Ala Pro Glu Lys
        675                 680                 685
AAC CCT GTG GAT GTG AAG GGG GAA GGA AAT GAG ACC ACC AAT ATG GTC 2112
Asn Pro Val Asp Val Lys Gly Glu Gly Asn Glu Thr Thr Asn Met Val
    690                 695                 700
ATC ACG TGG AAG CCG CTC CGG TGG ATG GAC TGG AAC GCC CCC CAG GTT 2160
Ile Thr Trp Lys Pro Leu Arg Trp Met Asp Trp Asn Ala Pro Gln Val
705                 710                 715                 720
CAG TAC CGC GTG CAG TGG CGC CCT CAG GGG ACA CGA GGG CCC TGG CAG 2208
Gln Tyr Arg Val Gln Trp Arg Pro Gln Gly Thr Arg Gly Pro Trp Gln
                725                 730                 735
GAG CAG ATT GTC AGC GAC CCC TTC CTG GTG GTG TCC AAC ACG TCC ACC 2256
Glu Gln Ile Val Ser Asp Pro Phe Leu Val Val Ser Asn Thr Ser Thr
            740                 745                 750
TTC GTG CCC TAT GAG ATC AAA GTC CAG GCC GTC AAC AGC CAG GGC AAG 2304
Phe Val Pro Tyr Glu Ile Lys Val Gln Ala Val Asn Ser Gln Gly Lys
        755                 760                 765
GGA CCA GAG CCC CAG GTC ACT ATC GGC TAC TCT GGA GAG GAC TAC CCC 2352
Gly Pro Glu Pro Gln Val Thr Ile Gly Tyr Ser Gly Glu Asp Tyr Pro
    770                 775                 780
CAG GCA ATC CCT GAG CTG GAA GGC ATT GAA ATC CTC AAC TCA AGT GCC 2400
Gln Ala Ile Pro Glu Leu Glu Gly Ile Glu Ile Leu Asn Ser Ser Ala
785                 790                 795                 800

GTG CTG GTC AAG TGG CGG CCG GTG GAC CTG GCC CAG GTC AAG GGC CAC 2448
Val Leu Val Lys Trp Arg Pro Val Asp Leu Ala Gln Val Lys Gly His
                805                 810                 815
CTC CGC GGA TAC AAT GTG ACG TAC TGG AGG GAG GGC AGT CAG AGG AAG 2496
Leu Arg Gly Tyr Asn Val Thr Tyr Trp Arg Glu Gly Ser Gln Arg Lys
            820                 825                 830
CAC AGC AAG AGA CAT ATC CAC AAA GAC CAT GTG GTG GTG CCC GCC AAC 2544
His Ser Lys Arg His Ile His Lys Asp His Val Val Val Pro Ala Asn
        835                 840                 845
ACC ACC AGT GTC ATC CTC AGT GGC TTG CGG CCC TAT AGC TCC TAC CAC 2592
Thr Thr Ser Val Ile Leu Ser Gly Leu Arg Pro Tyr Ser Ser Tyr His
    850                 855                 860
CTG GAG GTG CAG GCC TTT AAC GGG CGA GGA TCG GGG CCC GCC AGC GAG 2640
Leu Glu Val Gln Ala Phe Asn Gly Arg Gly Ser Gly Pro Ala Ser Glu
865                 870                 875                 880
TTC ACC TTC AGC ACC CCA GAG GGA GTG CCT GGC CAC CCC GAG GCG TTG 2688
Phe Thr Phe Ser Thr Pro Glu Gly Val Pro Gly His Pro Glu Ala Leu
                885                 890                 895
CAC CTG GAG TGC CAG TCG AAC ACC AGC CTG CTG CTG CGC TGG CAG CCC 2736
His Leu Glu Cys Gln Ser Asn Thr Ser Leu Leu Leu Arg Trp Gln Pro
            900                 905                 910
CCA CTC AGC CAC AAC GGC GTG CTC ACC GGC TAC GTG CTC TCC TAC CAC 2784
Pro Leu Ser His Asn Gly Val Leu Thr Gly Tyr Val Leu Ser Tyr His
        915                 920                 925

CCC CTG GAT GAG GGG GGC AAG GGG CAA CTG TCC TTC AAC CTT CGG GAC 2832
Pro Leu Asp Glu Gly Gly Lys Gly Gln Leu Ser Phe Asn Leu Arg Asp
    930                 935                 940
CCC GAA CTT CGG ACA CAC AAC CTG ACC GAT CTC AGC CCC CAC CTG CGG 2880
Pro Glu Leu Arg Thr His Asn Leu Thr Asp Leu Ser Pro His Leu Arg
945                 950                 955                 960
TAC CGC TTC CAG CTT CAG GCC ACC ACC AAA GAG GGC CCT GGT GAA GCC 2928
Tyr Arg Phe Gln Leu Gln Ala Thr Thr Lys Glu Gly Pro Gly Glu Ala
                965                 970                 975
ATC GTA CGG GAA GGA GGC ACT ATG GCC TTG TCT GGG ATC TCA GAT TTT 2976
Ile Val Arg Glu Gly Gly Thr Met Ala Leu Ser Gly Ile Ser Asp Phe
            980                 985                 990
GGC AAC ATC TCA GCC ACA GCG GGT GAA AAC TAC AGT GTC GTC TCC TGG 3024
Gly Asn Ile Ser Ala Thr Ala Gly Glu Asn Tyr Ser Val Val Ser Trp
        995                 1000                1005
GTC CCC AAG GAG GGC CAG TGC AAC TTC AGG TTC CAT ATC TTG TTC AAA 3072
Val Pro Lys Glu Gly Gln Cys Asn Phe Arg Phe His Ile Leu Phe Lys
    1010                1015                1020
GCC TTG GGA GAA GAG AAG GGT GGG GCT TCC CTT TCG CCA CAG TAT GTC 3120
Ala Leu Gly Glu Glu Lys Gly Gly Ala Ser Leu Ser Pro Gln Tyr Val
1025                1030                1035                1040
AGC TAC AAC CAG AGC TCC TAC ACG CAG TGG GAC CTG CAG CCT GAC ACT 3168
Ser Tyr Asn Gln Ser Ser Tyr Thr Gln Trp Asp Leu Gln Pro Asp Thr
                1045                1050                1055

GAC TAC GAG ATC CAC TTG TTT AAG GAG AGG ATG TTC CGG CAC CAA ATG 3216
Asp Tyr Glu Ile His Leu Phe Lys Glu Arg Met Phe Arg His Gln Met
            1060                1065                1070
GCT GTG AAG ACC AAT GGC ACA GGC CGC GTG AGG CTC CCT CCT GCT GGC 3264
Ala Val Lys Thr Asn Gly Thr Gly Arg Val Arg Leu Pro Pro Ala Gly
        1075                1080                1085
TTC GCC ACT GAG GGC TGG TTC ATC GGC TTT GTG AGT GCC ATC ATC CTC 3312
Phe Ala Thr Glu Gly Trp Phe Ile Gly Phe Val Ser Ala Ile Ile Leu
    1090                1095                1100
CTG CTC CTC GTC CTG CTC ATC CTC TGC TTC ATC AAG CGC AGC AAG GGC 3360
Leu Leu Leu Val Leu Leu Ile Leu Cys Phe Ile Lys Arg Ser Lys Gly
1105                1110                1115                1120
GGC AAA TAC TCA GTG AAG GAT AAG GAG GAC ACC CAG GTG GAC TCT GAG 3408
Gly Lys Tyr Ser Val Lys Asp Lys Glu Asp Thr Gln Val Asp Ser Glu
                1125                1130                1135
GCC CGA CCG ATG AAA GAT GAG ACC TTC GGC GAG TAC AGG TCC CTG GAG 3456
Ala Arg Pro Met Lys Asp Glu Thr Phe Gly Glu Tyr Arg Ser Leu Glu
            1140                1145                1150
AGT GAC AAC GAG GAG AAG GCC TTT GGC AGC AGC CAG CCA TCG CTC AAC 3504
Ser Asp Asn Glu Glu Lys Ala Phe Gly Ser Ser Gln Pro Ser Leu Asn
        1155                1160                1165
GGG GAC ATC AAG CCC CTG GGC AGT GAC GAC AGC CTG GCC GAT TAT GGG 3552
Gly Asp Ile Lys Pro Leu Gly Ser Asp Asp Ser Leu Ala Asp Tyr Gly
    1170                1175                1180

GGC AGC GTG GAT GTT CAG TTC AAC GAG GAT GGT TCG TTC ATT GGC CAG 3600
Gly Ser Val Asp Val Gln Phe Asn Glu Asp Gly Ser Phe Ile Gly Gln
1185                1190                1195                1200
TAC AGT GGC AAG AAG GAG AAG GAG GCG GCA GGG GGC AAT GAC AGC TCA 3648
Tyr Ser Gly Lys Lys Glu Lys Glu Ala Ala Gly Gly Asn Asp Ser Ser
                1205                1210                1215
GGG GCC ACT TCC CCC ATC AAC CTT GCC GTG GCC CTA GAA TAG 3690
Gly Ala Thr Ser Pro Ile Asn Leu Ala Val Ala Leu Glu
            1220                1225

### SEQ ID NO: 13

SEQUENCE LENGTH: 18
SEQUENCE TYPE: Nucleic acid
STRANDNESS: Single
TOPOLOGY: Linear
MOLECULE TYPE: Synthetic DNA
SEQUENCE:

AGGCTGATGT CATCTGTG 18

### SEQ ID NO: 14

SEQUENCE LENGTH: 18
SEQUENCE TYPE: Nucleic acid
STRANDNESS: Single
TOPOLOGY: Linear
MOLECULE TYPE: Synthetic DNA
SEQUENCE:

CGGAAGAGGG AGAAGGTG 18

### SEQ ID NO: 15

SEQUENCE LENGTH: 24
SEQUENCE TYPE: Nucleic acid
STRANDNESS: Single
TOPOLOGY: Linear
MOLECULE TYPE: Synthetic DNA
SEQUENCE:

TTCTCGAGAT GGTAGTGGTG CTGC 24

### SEQ ID NO: 16

SEQUENCE LENGTH: 22
SEQUENCE TYPE: Nucleic acid
STRANDNESS: Single
TOPOLOGY: Linear
MOLECULE TYPE: Synthetic DNA
SEQUENCE:

TCTTCCTTGG GTTTGAAGTG GA 22

### SEQ ID NO: 17

SEQUENCE LENGTH: 3759
SEQUENCE TYPE: Nucleic acid
STRANDNESS: Double
TOPOLOGY: Linear
MOLECULE TYPE: cDNA
SEQUENCE:

ATG GTA GTG GTG CTG CGG TAC GTG TGG CCT CTC CTC CTC TGC AGC CCC 48
Met Val Val Val Leu Arg Tyr Val Trp Pro Leu Leu Leu Cys Ser Pro
1               5                   10                  15

TGC CTG CTT ATC CAG ATC CCC GAG GAA TTG ATG GAG CCA CCT GTC ATC 96
Cys Leu Leu Ile Gln Ile Pro Glu Glu Leu Met Glu Pro Pro Val Ile
            20                  25                  30
ACG GAA CAG TCT CCA CGG CGC CTG GTT GTC TTC CCC ACG GAT GAC ATC 144
Thr Glu Gln Ser Pro Arg Arg Leu Val Val Phe Pro Thr Asp Asp Ile
        35                  40                  45
AGC CTC AAG TGT GAG GCC AGT GGC AAG CCC GAA GTG CAG TTC CGC TGG 192
Ser Leu Lys Cys Glu Ala Ser Gly Lys Pro Glu Val Gln Phe Arg Trp
    50                  55                  60
ACG AGG GAT GGT GTC CAC TTC AAA CCC AAG GAA GAG CTG GGT GTG ACC 240
Thr Arg Asp Gly Val His Phe Lys Pro Lys Glu Glu Leu Gly Val Thr
65                  70                  75                  80
GTG TAC CAG TCG CCC CAC TCT GGC TCC TTC ACC ATC ACG GGC AAC AAC 288
Val Tyr Gln Ser Pro His Ser Gly Ser Phe Thr Ile Thr Gly Asn Asn
                85                  90                  95
AGC AAC TTT GCT CAG AGG TTC CAG GGC ATC TAC CGC TGC TTT GCC AGC 336
Ser Asn Phe Ala Gln Arg Phe Gln Gly Ile Tyr Arg Cys Phe Ala Ser
            100                 105                 110
AAT AAG CTG GGC ACC GCC ATG TCC CAT GAG ATC CGG CTC ATG GCC GAG 384
Asn Lys Leu Gly Thr Ala Met Ser His Glu Ile Arg Leu Met Ala Glu
        115                 120                 125
GGT GCC CCC AAG TGG CCA AAG GAG ACA GTG AAG CCC GTG GAG GTG GAG 432
Gly Ala Pro Lys Trp Pro Lys Glu Thr Val Lys Pro Val Glu Val Glu
    130                 135                 140

GAA GGG GAG TCA GTG GTT CTG CCT TGC AAC CCT CCC CCA AGT GCA GAG     480
Glu Gly Glu Ser Val Val Leu Pro Cys Asn Pro Pro Pro Ser Ala Glu
145                 150                 155                 160
CCT CTC CGG ATC TAC TGG ATG AAC AGC AAG ATC TTG CAC ATC AAG CAG     528
Pro Leu Arg Ile Tyr Trp Met Asn Ser Lys Ile Leu His Ile Lys Gln
                165                 170                 175
GAC GAG CGG GTG ACG ATG GGC CAG AAC GGC AAC CTC TAC TTT GCC AAT     576
Asp Glu Arg Val Thr Met Gly Gln Asn Gly Asn Leu Tyr Phe Ala Asn
            180                 185                 190
GTG CTC ACC TCC GAC AAC CAC TCA GAC TAC ATC TGC CAC GCC CAC TTC     624
Val Leu Thr Ser Asp Asn His Ser Asp Tyr Ile Cys His Ala His Phe
        195                 200                 205
CCA GGC ACC AGG ACC ATC ATT CAG AAG GAA CCC ATT GAC CTC CGG GTC     672
Pro Gly Thr Arg Thr Ile Ile Gln Lys Glu Pro Ile Asp Leu Arg Val
    210                 215                 220
AAG GCC ACC AAC AGC ATG ATT GAC AGG AAG CCG CGC CTG CTC TTC CCC     720
Lys Ala Thr Asn Ser Met Ile Asp Arg Lys Pro Arg Leu Leu Phe Pro
225                 230                 235                 240
ACC AAC TCC AGC AGC CAC CTG GTG GCC TTG CAG GGG CAG CCA TTG GTC     768
Thr Asn Ser Ser Ser His Leu Val Ala Leu Gln Gly Gln Pro Leu Val
                245                 250                 255
CTG GAG TGC ATC GCC GAG GGC TTT CCC ACG CCC ACC ATC AAA TGG CTG     816
Leu Glu Cys Ile Ala Glu Gly Phe Pro Thr Pro Thr Ile Lys Trp Leu
            260                 265                 270

CGC CCC AGT GGC CCC ATG CCA GCC GAC CGT GTC ACC TAC CAG AAC CAC     864
Arg Pro Ser Gly Pro Met Pro Ala Asp Arg Val Thr Tyr Gln Asn His
        275                 280                 285
AAC AAG ACC CTG CAG CTG CTG AAA GTG GGC GAG GAG GAT GAT GGC GAG     912
Asn Lys Thr Leu Gln Leu Leu Lys Val Gly Glu Glu Asp Asp Gly Glu
    290                 295                 300
TAC CGC TGC CTG GCC GAG AAC TCA CTG GGC AGT GCC CGG CAT GCG TAC     960
Tyr Arg Cys Leu Ala Glu Asn Ser Leu Gly Ser Ala Arg His Ala Tyr
305                 310                 315                 320
TAT GTC ACC GTG GAG GCT GCC CCG TAC TGG CTG CAC AAG CCC CAG AGC    1008
Tyr Val Thr Val Glu Ala Ala Pro Tyr Trp Leu His Lys Pro Gln Ser
                325                 330                 335
CAT CTA TAT GGG CCA GGA GAG ACT GCC CGC CTG GAC TGC CAA GTC CAG    1056
His Leu Tyr Gly Pro Gly Glu Thr Ala Arg Leu Asp Cys Gln Val Gln
            340                 345                 350
GGC AGG CCC CAA CCA GAG GTC ACC TGG AGA ATC AAC GGG ATC CCT GTG    1104
Gly Arg Pro Gln Pro Glu Val Thr Trp Arg Ile Asn Gly Ile Pro Val
        355                 360                 365
GAG GAG CTG GCC AAA GAC CAG AAG TAC CGG ATT CAG CTG GGC GCC CTG    1152
Glu Glu Leu Ala Lys Asp Gln Lys Tyr Arg Ile Gln Leu Gly Ala Leu
    370                 375                 380
ATC CTG AGC AAC GTG CAG CCC AGT GAC ACA ATG GTG ACC CAA TGT GAG    1200
Ile Leu Ser Asn Val Gln Pro Ser Asp Thr Met Val Thr Gln Cys Glu
385                 390                 395                 400

GCC CGC AAC CGG CAC GGG CTC TTG CTG GCC AAT GCC TAC ATC TAC GTT 1248
Ala Arg Asn Arg His Gly Leu Leu Leu Ala Asn Ala Tyr Ile Tyr Val
                405                 410                 415
GTC CAG CTG CCA GCC AAG ATC CTG ACT GCG GAC AAT CAG ACG TAC ATG 1296
Val Gln Leu Pro Ala Lys Ile Leu Thr Ala Asp Asn Gln Thr Tyr Met
            420                 425                 430
GCT GTC CAG GGC AGC ACT GCC TAC CTT CTG TGC AAG GCC TTC GGA GCG 1344
Ala Val Gln Gly Ser Thr Ala Tyr Leu Leu Cys Lys Ala Phe Gly Ala
        435                 440                 445
CCT GTG CCC AGT GTT CAG TGG CTG GAC GAG GAT GGG ACA ACA GTG CTT 1392
Pro Val Pro Ser Val Gln Trp Leu Asp Glu Asp Gly Thr Thr Val Leu
    450                 455                 460
CAG GAC GAA CGC TTC TTC CCC TAT GCC AAT GGG ACC CTG GGC ATT CGA 1440
Gln Asp Glu Arg Phe Phe Pro Tyr Ala Asn Gly Thr Leu Gly Ile Arg
465                 470                 475                 480
GAC CTC CAG GCC AAT GAC ACC GGA CGC TAC TTC TGC CTG GCT GCC AAT 1488
Asp Leu Gln Ala Asn Asp Thr Gly Arg Tyr Phe Cys Leu Ala Ala Asn
                485                 490                 495
GAC CAA AAC AAT GTT ACC ATC ATG GCT AAC CTG AAG GTT AAA GAT GCA 1536
Asp Gln Asn Asn Val Thr Ile Met Ala Asn Leu Lys Val Lys Asp Ala
            500                 505                 510
ACT CAG ATC ACT CAG GGG CCC CGC AGC ACA ATC GAG AAG AAA GGT TCC 1584
Thr Gln Ile Thr Gln Gly Pro Arg Ser Thr Ile Glu Lys Lys Gly Ser
        515                 520                 525

AGG GTG ACC TTC ACG TGC CAG GCC TCC TTT GAC CCC TCC TTG CAG CCC   1632
Arg Val Thr Phe Thr Cys Gln Ala Ser Phe Asp Pro Ser Leu Gln Pro
    530                 535                 540
AGC ATC ACC TGG CGT GGG GAC GGT CGA GAC CTC CAG GAG CTT GGG GAC   1680
Ser Ile Thr Trp Arg Gly Asp Gly Arg Asp Leu Gln Glu Leu Gly Asp
545                 550                 555                 560
AGT GAC AAG TAC TTC ATA GAG GAT GGG CGC CTG GTC ATC CAC AGC CTG   1728
Ser Asp Lys Tyr Phe Ile Glu Asp Gly Arg Leu Val Ile His Ser Leu
                565                 570                 575
GAC TAC AGC GAC CAG GGC AAC TAC AGC TGC GTG GCC AGT ACC GAA CTG   1776
Asp Tyr Ser Asp Gln Gly Asn Tyr Ser Cys Val Ala Ser Thr Glu Leu
            580                 585                 590
GAT GTG GTG GAG AGT AGG GCA CAG CTC TTG GTG GTG GGG AGC CCT GGG   1824
Asp Val Val Glu Ser Arg Ala Gln Leu Leu Val Val Gly Ser Pro Gly
        595                 600                 605
CCG GTG CCA CGG CTG GTG CTG TCC GAC CTG CAC CTC GTG ACG CAG AGC   1872
Pro Val Pro Arg Leu Val Leu Ser Asp Leu His Leu Val Thr Gln Ser
    610                 615                 620
CAG GTG CGC GTG TCC TGG AGT CCT GCA GAA GAC CAC AAT GCC CCC ATT   1920
Gln Val Arg Val Ser Trp Ser Pro Ala Glu Asp His Asn Ala Pro Ile
625                 630                 635                 640
GAG AAA TAT GAC ATT GAA TTT GAG GAC AAG GAA ATG GCG CCT GAA AAA   1968
Glu Lys Tyr Asp Ile Glu Phe Glu Asp Lys Glu Met Ala Pro Glu Lys
                645                 650                 655

TGG TAC AGT CTG GGC AAG GTT CCA GGG AAC CAG ACC TCT ACC ACC CTC 2016
Trp Tyr Ser Leu Gly Lys Val Pro Gly Asn Gln Thr Ser Thr Thr Leu
            660                 665                 670
AAG CTG TCG CCC TAT GTC CAC TAC ACC TTT AGG GTT ACT GCC ATA AAC 2064
Lys Leu Ser Pro Tyr Val His Tyr Thr Phe Arg Val Thr Ala Ile Asn
        675                 680                 685
AAA TAT GGC CCC GGG GAG CCC AGC CCG GTC TCT GAG ACT GTG GTC ACA 2112
Lys Tyr Gly Pro Gly Glu Pro Ser Pro Val Ser Glu Thr Val Val Thr
    690                 695                 700
CCT GAG GCA GCC CCA GAG AAG AAC CCT GTG GAT GTG AAG GGG GAA GGA 2160
Pro Glu Ala Ala Pro Glu Lys Asn Pro Val Asp Val Lys Gly Glu Gly
705                 710                 715                 720
AAT GAG ACC ACC AAT ATG GTC ATC ACG TGG AAG CCG CTC CGG TGG ATG 2208
Asn Glu Thr Thr Asn Met Val Ile Thr Trp Lys Pro Leu Arg Trp Met
                725                 730                 735
GAC TGG AAC GCC CCC CAG GTT CAG TAC CGC GTG CAG TGG CGC CCT CAG 2256
Asp Trp Asn Ala Pro Gln Val Gln Tyr Arg Val Gln Trp Arg Pro Gln
            740                 745                 750
GGG ACA CGA GGG CCC TGG CAG GAG CAG ATT GTC AGC GAC CCC TTC CTG 2304
Gly Thr Arg Gly Pro Trp Gln Glu Gln Ile Val Ser Asp Pro Phe Leu
        755                 760                 765
GTG GTG TCC AAC ACG TCC ACC TTC GTG CCC TAT GAG ATC AAA GTC CAG 2352
Val Val Ser Asn Thr Ser Thr Phe Val Pro Tyr Glu Ile Lys Val Gln
    770                 775                 780

GCC GTC AAC AGC CAG GGC AAG GGA CCA GAG CCC CAG GTC ACT ATC GGC 2400
Ala Val Asn Ser Gln Gly Lys Gly Pro Glu Pro Gln Val Thr Ile Gly
785                 790                 795                 800
TAC TCT GGA GAG GAC TAC CCC CAG GCA ATC CCT GAG CTG GAA GGC ATT 2448
Tyr Ser Gly Glu Asp Tyr Pro Gln Ala Ile Pro Glu Leu Glu Gly Ile
                805                 810                 815
GAA ATC CTC AAC TCA AGT GCC GTG CTG GTC AAG TGG CGG CCG GTG GAC 2496
Glu Ile Leu Asn Ser Ser Ala Val Leu Val Lys Trp Arg Pro Val Asp
            820                 825                 830
CTG GCC CAG GTC AAG GGC CAC CTC CGC GGA TAC AAT GTG ACG TAC TGG 2544
Leu Ala Gln Val Lys Gly His Leu Arg Gly Tyr Asn Val Thr Tyr Trp
        835                 840                 845
AGG GAG GGC AGT CAG AGG AAG CAC AGC AAG AGA CAT ATC CAC AAA GAC 2592
Arg Glu Gly Ser Gln Arg Lys His Ser Lys Arg His Ile His Lys Asp
    850                 855                 860
CAT GTG GTG GTG CCC GCC AAC ACC ACC AGT GTC ATC CTC AGT GGC TTG 2640
His Val Val Val Pro Ala Asn Thr Thr Ser Val Ile Leu Ser Gly Leu
865                 870                 875                 880
CGG CCC TAT AGC TCC TAC CAC CTG GAG GTG CAG GCC TTT AAC GGG CGA 2688
Arg Pro Tyr Ser Ser Tyr His Leu Glu Val Gln Ala Phe Asn Gly Arg
                885                 890                 895
GGA TCG GGG CCC GCC AGC GAG TTC ACC TTC AGC ACC CCA GAG GGA GTG 2736
Gly Ser Gly Pro Ala Ser Glu Phe Thr Phe Ser Thr Pro Glu Gly Val
            900                 905                 910

CCT GGC CAC CCC GAG GCG TTG CAC CTG GAG TGC CAG TCG AAC ACC AGC 2784
Pro Gly His Pro Glu Ala Leu His Leu Glu Cys Gln Ser Asn Thr Ser
        915                 920                 925
CTG CTG CTG CGC TGG CAG CCC CCA CTC AGC CAC AAC GGC GTG CTC ACC 2832
Leu Leu Leu Arg Trp Gln Pro Pro Leu Ser His Asn Gly Val Leu Thr
    930                 935                 940
GGC TAC GTG CTC TCC TAC CAC CCC CTG GAT GAG GGG GGC AAG GGG CAA 2880
Gly Tyr Val Leu Ser Tyr His Pro Leu Asp Glu Gly Gly Lys Gly Gln
945                 950                 955                 960
CTG TCC TTC AAC CTT CGG GAC CCC GAA CTT CGG ACA CAC AAC CTG ACC 2928
Leu Ser Phe Asn Leu Arg Asp Pro Glu Leu Arg Thr His Asn Leu Thr
                965                 970                 975
GAT CTC AGC CCC CAC CTG CGG TAC CGC TTC CAG CTT CAG GCC ACC ACC 2976
Asp Leu Ser Pro His Leu Arg Tyr Arg Phe Gln Leu Gln Ala Thr Thr
            980                 985                 990
AAA GAG GGC CCT GGT GAA GCC ATC GTA CGG GAA GGA GGC ACT ATG GCC 3024
Lys Glu Gly Pro Gly Glu Ala Ile Val Arg Glu Gly Gly Thr Met Ala
        995                 1000                1005
TTG TCT GGG ATC TCA GAT TTT GGC AAC ATC TCA GCC ACA GCG GGT GAA 3072
Leu Ser Gly Ile Ser Asp Phe Gly Asn Ile Ser Ala Thr Ala Gly Glu
    1010                1015                1020
AAC TAC AGT GTC GTC TCC TGG GTC CCC AAG GAG GGC CAG TGC AAC TTC 3120
Asn Tyr Ser Val Val Ser Trp Val Pro Lys Glu Gly Gln Cys Asn Phe
1025                1030                1035                1040

AGG TTC CAT ATC TTG TTC AAA GCC TTG GGA GAA GAG AAG GGT GGG GCT 3168
Arg Phe His Ile Leu Phe Lys Ala Leu Gly Glu Glu Lys Gly Gly Ala
                1045                1050                1055
TCC CTT TCG CCA CAG TAT GTC AGC TAC AAC CAG AGC TCC TAC ACG CAG 3216
Ser Leu Ser Pro Gln Tyr Val Ser Tyr Asn Gln Ser Ser Tyr Thr Gln
            1060                1065                1070
TGG GAC CTG CAG CCT GAC ACT GAC TAC GAG ATC CAC TTG TTT AAG GAG 3264
Trp Asp Leu Gln Pro Asp Thr Asp Tyr Glu Ile His Leu Phe Lys Glu
        1075                1080                1085
AGG ATG TTC CGG CAC CAA ATG GCT GTG AAG ACC AAT GGC ACA GGC CGC 3312
Arg Met Phe Arg His Gln Met Ala Val Lys Thr Asn Gly Thr Gly Arg
    1090                1095                1100
GTG AGG CTC CCT CCT GCT GGC TTC GCC ACT GAG GGC TGG TTC ATC GGC 3360
Val Arg Leu Pro Pro Ala Gly Phe Ala Thr Glu Gly Trp Phe Ile Gly
1105                1110                1115                1120
TTT GTG AGT GCC ATC ATC CTC CTG CTC CTC GTC CTG CTC ATC CTC TGC 3408
Phe Val Ser Ala Ile Ile Leu Leu Leu Leu Val Leu Leu Ile Leu Cys
                1125                1130                1135
TTC ATC AAG CGC AGC AAG GGC GGC AAA TAC TCA GTG AAG GAT AAG GAG 3456
Phe Ile Lys Arg Ser Lys Gly Gly Lys Tyr Ser Val Lys Asp Lys Glu
            1140                1145                1150
GAC ACC CAG GTG GAC TCT GAG GCC CGA CCG ATG AAA GAT GAG ACC TTC 3504
Asp Thr Gln Val Asp Ser Glu Ala Arg Pro Met Lys Asp Glu Thr Phe
        1155                1160                1165

GGC GAG TAC AGG TCC CTG GAG AGT GAC AAC GAG GAG AAG GCC TTT GGC   3552
Gly Glu Tyr Arg Ser Leu Glu Ser Asp Asn Glu Glu Lys Ala Phe Gly
    1170                1175                1180
AGC AGC CAG CCA TCG CTC AAC GGG GAC ATC AAG CCC CTG GGC AGT GAC   3600
Ser Ser Gln Pro Ser Leu Asn Gly Asp Ile Lys Pro Leu Gly Ser Asp
1185                1190                1195                1200
GAC AGC CTG GCC GAT TAT GGG GGC AGC GTG GAT GTT CAG TTC AAC GAG   3648
Asp Ser Leu Ala Asp Tyr Gly Gly Ser Val Asp Val Gln Phe Asn Glu
                1205                1210                1215
GAT GGT TCG TTC ATT GGC CAG TAC AGT GGC AAG AAG GAG AAG GAG GCG   3696
Asp Gly Ser Phe Ile Gly Gln Tyr Ser Gly Lys Lys Glu Lys Glu Ala
            1220                1225                1230
GCA GGG GGC AAT GAC AGC TCA GGG GCC ACT TCC CCC ATC AAC CTT GCC   3744
Ala Gly Gly Asn Asp Ser Ser Gly Ala Thr Ser Pro Ile Asn Leu Ala
        1235                1240                1245
GTG GCC CTA GAA TAG                                               3759
Val Ala Leu Glu
    1250

### SEQ ID NO: 18

SEQUENCE LENGTH: 3774
SEQUENCE TYPE: Nucleic acid
STRANDNESS: Double
TOPOLOGY: Linear
MOLECULE TYPE: cDNA
SEQUENCE:

ATG GTA GTG GTG CTG CGG TAC GTG TGG CCT CTC CTC CTC TGC AGC CCC 48
Met Val Val Val Leu Arg Tyr Val Trp Pro Leu Leu Leu Cys Ser Pro
1               5                   10                  15
TGC CTG CTT ATC CAG ATC CCC GAG GAA TAT GAA GGA CAC CAT GTG ATG 96
Cys Leu Leu Ile Gln Ile Pro Glu Glu Tyr Gly Gly His His Val Met
            20                  25                  30
GAG CCA CCT GTC ATC ACG GAA CAG TCT CCA CGG CGC CTG GTT GTC TTC 144
Glu Pro Pro Val Ile Thr Glu Gln Ser Pro Arg Arg Leu Val Val Phe
        35                  40                  45
CCC ACG GAT GAC ATC AGC CTC AAG TGT GAG GCC AGT GGC AAG CCC GAA 192
Pro Thr Asp Asp Ile Ser Leu Lys Cys Glu Ala Ser Gly Lys Pro Glu
    50                  55                  60
GTG CAG TTC CGC TGG ACG AGG GAT GGT GTC CAC TTC AAA CCC AAG GAA 240
Val Gln Phe Arg Trp Thr Arg Asp Gly Val His Phe Lys Pro Lys Glu
65                  70                  75                  80
GAG CTG GGT GTG ACC GTG TAC CAG TCG CCC CAC TCT GGC TCC TTC ACC 288
Glu Leu Gly Val Thr Val Tyr Gln Ser Pro His Ser Gly Ser Phe Thr
                85                  90                  95
ATC ACG GGC AAC AAC AGC AAC TTT GCT CAG AGG TTC CAG GGC ATC TAC 336
Ile Thr Gly Asn Asn Ser Asn Phe Ala Gln Arg Phe Gln Gly Ile Tyr
            100                 105                 110
CGC TGC TTT GCC AGC AAT AAG CTG GGC ACC GCC ATG TCC CAT GAG ATC 384
Arg Cys Phe Ala Ser Asn Lys Leu Gly Thr Ala Met Ser His Glu Ile
        115                 120                 125

CGG CTC ATG GCC GAG GGT GCC CCC AAG TGG CCA AAG GAG ACA GTG AAG 432
Arg Leu Met Ala Glu Gly Ala Pro Lys Trp Pro Lys Glu Thr Val Lys
    130                 135                 140
CCC GTG GAG GTG GAG GAA GGG GAG TCA GTG GTT CTG CCT TGC AAC CCT 480
Pro Val Glu Val Glu Glu Gly Glu Ser Val Val Leu Pro Cys Asn Pro
145                 150                 155                 160
CCC CCA AGT GCA GAG CCT CTC CGG ATC TAC TGG ATG AAC AGC AAG ATC 528
Pro Pro Ser Ala Glu Pro Leu Arg Ile Tyr Trp Met Asn Ser Lys Ile
                165                 170                 175
TTG CAC ATC AAG CAG GAC GAG CGG GTG ACG ATG GGC CAG AAC GGC AAC 576
Leu His Ile Lys Gln Asp Glu Arg Val Thr Met Gly Gln Asn Gly Asn
            180                 185                 190
CTC TAC TTT GCC AAT GTG CTC ACC TCC GAC AAC CAC TCA GAC TAC ATC 624
Leu Tyr Phe Ala Asn Val Leu Thr Ser Asp Asn His Ser Asp Tyr Ile
        195                 200                 205
TGC CAC GCC CAC TTC CCA GGC ACC AGG ACC ATC ATT CAG AAG GAA CCC 672
Cys His Ala His Phe Pro Gly Thr Arg Thr Ile Ile Gln Lys Glu Pro
    210                 215                 220
ATT GAC CTC CGG GTC AAG GCC ACC AAC AGC ATG ATT GAC AGG AAG CCG 720
Ile Asp Leu Arg Val Lys Ala Thr Asn Ser Met Ile Asp Arg Lys Pro
225                 230                 235                 240
CGC CTG CTC TTC CCC ACC AAC TCC AGC AGC CAC CTG GTG GCC TTG CAG 768
Arg Leu Leu Phe Pro Thr Asn Ser Ser Ser His Leu Val Ala Leu Gln
                245                 250                 255

GGG CAG CCA TTG GTC CTG GAG TGC ATC GCC GAG GGC TTT CCC ACG CCC 816
Gly Gln Pro Leu Val Leu Glu Cys Ile Ala Glu Gly Phe Pro Thr Pro
            260                 265                 270
ACC ATC AAA TGG CTG CGC CCC AGT GGC CCC ATG CCA GCC GAC CGT GTC 864
Thr Ile Lys Trp Leu Arg Pro Ser Gly Pro Met Pro Ala Asp Arg Val
        275                 280                 285
ACC TAC CAG AAC CAC AAC AAG ACC CTG CAG CTG CTG AAA GTG GGC GAG 912
Thr Tyr Gln Asn His Asn Lys Thr Leu Gln Leu Leu Lys Val Gly Glu
    290                 295                 300
GAG GAT GAT GGC GAG TAC CGC TGC CTG GCC GAG AAC TCA CTG GGC AGT 960
Glu Asp Asp Gly Glu Tyr Arg Cys Leu Ala Glu Asn Ser Leu Gly Ser
305                 310                 315                 320
GCC CGG CAT GCG TAC TAT GTC ACC GTG GAG GCT GCC CCG TAC TGG CTG 1008
Ala Arg His Ala Tyr Tyr Val Thr Val Glu Ala Ala Pro Tyr Trp Leu
                325                 330                 335
CAC AAG CCC CAG AGC CAT CTA TAT GGG CCA GGA GAG ACT GCC CGC CTG 1056
His Lys Pro Gln Ser His Leu Tyr Gly Pro Gly Glu Thr Ala Arg Leu
            340                 345                 350
GAC TGC CAA GTC CAG GGC AGG CCC CAA CCA GAG GTC ACC TGG AGA ATC 1104
Asp Cys Gln Val Gln Gly Arg Pro Gln Pro Glu Val Thr Trp Arg Ile
        355                 360                 365
AAC GGG ATC CCT GTG GAG GAG CTG GCC AAA GAC CAG AAG TAC CGG ATT 1152
Asn Gly Ile Pro Val Glu Glu Leu Ala Lys Asp Gln Lys Tyr Arg Ile
    370                 375                 380

CAG CTG GGC GCC CTG ATC CTG AGC AAC GTG CAG CCC AGT GAC ACA ATG 1200
Gln Leu Gly Ala Leu Ile Leu Ser Asn Val Gln Pro Ser Asp Thr Met
385                 390                 395                 400
GTG ACC CAA TGT GAG GCC CGC AAC CGG CAC GGG CTC TTG CTG GCC AAT 1248
Val Thr Gln Cys Glu Ala Arg Asn Arg His Gly Leu Leu Leu Ala Asn
                405                 410                 415
GCC TAC ATC TAC GTT GTC CAG CTG CCA GCC AAG ATC CTG ACT GCG GAC 1296
Ala Tyr Ile Tyr Val Val Gln Leu Pro Ala Lys Ile Leu Thr Ala Asp
            420                 425                 430
AAT CAG ACG TAC ATG GCT GTC CAG GGC AGC ACT GCC TAC CTT CTG TGC 1344
Asn Gln Thr Tyr Met Ala Val Gln Gly Ser Thr Ala Tyr Leu Leu Cys
        435                 440                 445
AAG GCC TTC GGA GCG CCT GTG CCC AGT GTT CAG TGG CTG GAC GAG GAT 1392
Lys Ala Phe Gly Ala Pro Val Pro Ser Val Gln Trp Leu Asp Glu Asp
    450                 455                 460
GGG ACA ACA GTG CTT CAG GAC GAA CGC TTC TTC CCC TAT GCC AAT GGG 1440
Gly Thr Thr Val Leu Gln Asp Glu Arg Phe Phe Pro Tyr Ala Asn Gly
465                 470                 475                 480
ACC CTG GGC ATT CGA GAC CTC CAG GCC AAT GAC ACC GGA CGC TAC TTC 1488
Thr Leu Gly Ile Arg Asp Leu Gln Ala Asn Asp Thr Gly Arg Tyr Phe
                485                 490                 495
TGC CTG GCT GCC AAT GAC CAA AAC AAT GTT ACC ATC ATG GCT AAC CTG 1536
Cys Leu Ala Ala Asn Asp Gln Asn Asn Val Thr Ile Met Ala Asn Leu
            500                 505                 510

AAG GTT AAA GAT GCA ACT CAG ATC ACT CAG GGG CCC CGC AGC ACA ATC 1584
Lys Val Lys Asp Ala Thr Gln Ile Thr Gln Gly Pro Arg Ser Thr Ile
        515                 520                 525
GAG AAG AAA GGT TCC AGG GTG ACC TTC ACG TGC CAG GCC TCC TTT GAC 1632
Glu Lys Lys Gly Ser Arg Val Thr Phe Thr Cys Gln Ala Ser Phe Asp
    530                 535                 540
CCC TCC TTG CAG CCC AGC ATC ACC TGG CGT GGG GAC GGT CGA GAC CTC 1680
Pro Ser Leu Gln Pro Ser Ile Thr Trp Arg Gly Asp Gly Arg Asp Leu
545                 550                 555                 560
CAG GAG CTT GGG GAC AGT GAC AAG TAC TTC ATA GAG GAT GGG CGC CTG 1728
Gln Glu Leu Gly Asp Ser Asp Lys Tyr Phe Ile Glu Asp Gly Arg Leu
                565                 570                 575
GTC ATC CAC AGC CTG GAC TAC AGC GAC CAG GGC AAC TAC AGC TGC GTG 1776
Val Ile His Ser Leu Asp Tyr Ser Asp Gln Gly Asn Tyr Ser Cys Val
            580                 585                 590
GCC AGT ACC GAA CTG GAT GTG GTG GAG AGT AGG GCA CAG CTC TTG GTG 1824
Ala Ser Thr Glu Leu Asp Val Val Glu Ser Arg Ala Gln Leu Leu Val
        595                 600                 605
GTG GGG AGC CCT GGG CCG GTG CCA CGG CTG GTG CTG TCC GAC CTG CAC 1872
Val Gly Ser Pro Gly Pro Val Pro Arg Leu Val Leu Ser Asp Leu His
    610                 615                 620
CTC GTG ACG CAG AGC CAG GTG CGC GTG TCC TGG AGT CCT GCA GAA GAC 1920
Leu Val Thr Gln Ser Gln Val Arg Val Ser Trp Ser Pro Ala Glu Asp
625                 630                 635                 640

CAC AAT GCC CCC ATT GAG AAA TAT GAC ATT GAA TTT GAG GAC AAG GAA 1968
His Asn Ala Pro Ile Glu Lys Tyr Asp Ile Glu Phe Glu Asp Lys Glu
                645                 650                 655
ATG GCG CCT GAA AAA TGG TAC AGT CTG GGC AAG GTT CCA GGG AAC CAG 2016
Met Ala Pro Glu Lys Trp Tyr Ser Leu Gly Lys Val Pro Gly Asn Gln
            660                 665                 670
ACC TCT ACC ACC CTC AAG CTG TCG CCC TAT GTC CAC TAC ACC TTT AGG 2064
Thr Ser Thr Thr Leu Lys Leu Ser Pro Tyr Val His Tyr Thr Phe Arg
        675                 680                 685
GTT ACT GCC ATA AAC AAA TAT GGC CCC GGG GAG CCC AGC CCG GTC TCT 2112
Val Thr Ala Ile Asn Lys Tyr Gly Pro Gly Glu Pro Ser Pro Val Ser
    690                 695                 700
GAG ACT GTG GTC ACA CCT GAG GCA GCC CCA GAG AAG AAC CCT GTG GAT 2160
Glu Thr Val Val Thr Pro Glu Ala Ala Pro Glu Lys Asn Pro Val Asp
705                 710                 715                 720
GTG AAG GGG GAA GGA AAT GAG ACC ACC AAT ATG GTC ATC ACG TGG AAG 2208
Val Lys Gly Glu Gly Asn Glu Thr Thr Asn Met Val Ile Thr Trp Lys
                725                 730                 735
CCG CTC CGG TGG ATG GAC TGG AAC GCC CCC CAG GTT CAG TAC CGC GTG 2256
Pro Leu Arg Trp Met Asp Trp Asn Ala Pro Gln Val Gln Tyr Arg Val
            740                 745                 750
CAG TGG CGC CCT CAG GGG ACA CGA GGG CCC TGG CAG GAG CAG ATT GTC 2304
Gln Trp Arg Pro Gln Gly Thr Arg Gly Pro Trp Gln Glu Gln Ile Val
        755                 760                 765

AGC GAC CCC TTC CTG GTG GTG TCC AAC ACG TCC ACC TTC GTG CCC TAT 2352
Ser Asp Pro Phe Leu Val Val Ser Asn Thr Ser Thr Phe Val Pro Tyr
    770                 775                 780
GAG ATC AAA GTC CAG GCC GTC AAC AGC CAG GGC AAG GGA CCA GAG CCC 2400
Glu Ile Lys Val Gln Ala Val Asn Ser Gln Gly Lys Gly Pro Glu Pro
785                 790                 795                 800
CAG GTC ACT ATC GGC TAC TCT GGA GAG GAC TAC CCC CAG GCA ATC CCT 2448
Gln Val Thr Ile Gly Tyr Ser Gly Glu Asp Tyr Pro Gln Ala Ile Pro
                805                 810                 815
GAG CTG GAA GGC ATT GAA ATC CTC AAC TCA AGT GCC GTG CTG GTC AAG 2496
Glu Leu Glu Gly Ile Glu Ile Leu Asn Ser Ser Ala Val Leu Val Lys
            820                 825                 830
TGG CGG CCG GTG GAC CTG GCC CAG GTC AAG GGC CAC CTC CGC GGA TAC 2544
Trp Arg Pro Val Asp Leu Ala Gln Val Lys Gly His Leu Arg Gly Tyr
        835                 840                 845
AAT GTG ACG TAC TGG AGG GAG GGC AGT CAG AGG AAG CAC AGC AAG AGA 2592
Asn Val Thr Tyr Trp Arg Glu Gly Ser Gln Arg Lys His Ser Lys Arg
    850                 855                 860
CAT ATC CAC AAA GAC CAT GTG GTG GTG CCC GCC AAC ACC ACC AGT GTC 2640
His Ile His Lys Asp His Val Val Val Pro Ala Asn Thr Thr Ser Val
865                 870                 875                 880
ATC CTC AGT GGC TTG CGG CCC TAT AGC TCC TAC CAC CTG GAG GTG CAG 2688
Ile Leu Ser Gly Leu Arg Pro Tyr Ser Ser Tyr His Leu Glu Val Gln
                885                 890                 895

GCC TTT AAC GGG CGA GGA TCG GGG CCC GCC AGC GAG TTC ACC TTC AGC 2736
Ala Phe Asn Gly Arg Gly Ser Gly Pro Ala Ser Glu Phe Thr Phe Ser
            900                 905                 910
ACC CCA GAG GGA GTG CCT GGC CAC CCC GAG GCG TTG CAC CTG GAG TGC 2784
Thr Pro Glu Gly Val Pro Gly His Pro Glu Ala Leu His Leu Glu Cys
        915                 920                 925
CAG TCG AAC ACC AGC CTG CTG CTG CGC TGG CAG CCC CCA CTC AGC CAC 2832
Gln Ser Asn Thr Ser Leu Leu Leu Arg Trp Gln Pro Pro Leu Ser His
    930                 935                 940
AAC GGC GTG CTC ACC GGC TAC GTG CTC TCC TAC CAC CCC CTG GAT GAG 2880
Asn Gly Val Leu Thr Gly Tyr Val Leu Ser Tyr His Pro Leu Asp Glu
945                 950                 955                 960
GGG GGC AAG GGG CAA CTG TCC TTC AAC CTT CGG GAC CCC GAA CTT CGG 2928
Gly Gly Lys Gly Gln Leu Ser Phe Asn Leu Arg Asp Pro Glu Leu Arg
                965                 970                 975
ACA CAC AAC CTG ACC GAT CTC AGC CCC CAC CTG CGG TAC CGC TTC CAG 2976
Thr His Asn Leu Thr Asp Leu Ser Pro His Leu Arg Tyr Arg Phe Gln
            980                 985                 990
CTT CAG GCC ACC ACC AAA GAG GGC CCT GGT GAA GCC ATC GTA CGG GAA 3024
Leu Gln Ala Thr Thr Lys Glu Gly Pro Gly Glu Ala Ile Val Arg Glu
        995                 1000                1005
GGA GGC ACT ATG GCC TTG TCT GGG ATC TCA GAT TTT GGC AAC ATC TCA 3072
Gly Gly Thr Met Ala Leu Ser Gly Ile Ser Asp Phe Gly Asn Ile Ser
    1010                1015                1020

GCC ACA GCG GGT GAA AAC TAC AGT GTC GTC TCC TGG GTC CCC AAG GAG 3120
Ala Thr Ala Gly Glu Asn Tyr Ser Val Val Ser Trp Val Pro Lys Glu
1025                1030                1035                1040
GGC CAG TGC AAC TTC AGG TTC CAT ATC TTG TTC AAA GCC TTG GGA GAA 3168
Gly Gln Cys Asn Phe Arg Phe His Ile Leu Phe Lys Ala Leu Gly Glu
                1045                1050                1055
GAG AAG GGT GGG GCT TCC CTT TCG CCA CAG TAT GTC AGC TAC AAC CAG 3216
Glu Lys Gly Gly Ala Ser Leu Ser Pro Gln Tyr Val Ser Tyr Asn Gln
            1060                1065                1070
AGC TCC TAC ACG CAG TGG GAC CTG CAG CCT GAC ACT GAC TAC GAG ATC 3264
Ser Ser Tyr Thr Gln Trp Asp Leu Gln Pro Asp Thr Asp Tyr Glu Ile
        1075                1080                1085
CAC TTG TTT AAG GAG AGG ATG TTC CGG CAC CAA ATG GCT GTG AAG ACC 3312
His Leu Phe Lys Glu Arg Met Phe Arg His Gln Met Ala Val Lys Thr
    1090                1095                1100
AAT GGC ACA GGC CGC GTG AGG CTC CCT CCT GCT GGC TTC GCC ACT GAG 3360
Asn Gly Thr Gly Arg Val Arg Leu Pro Pro Ala Gly Phe Ala Thr Glu
1105                1110                1115                1120
GGC TGG TTC ATC GGC TTT GTG AGT GCC ATC ATC CTC CTG CTC CTC GTC 3408
Gly Trp Phe Ile Gly Phe Val Ser Ala Ile Ile Leu Leu Leu Leu Val
                1125                1130                1135
CTG CTC ATC CTC TGC TTC ATC AAG CGC AGC AAG GGC GGC AAA TAC TCA 3456
Leu Leu Ile Leu Cys Phe Ile Lys Arg Ser Lys Gly Gly Lys Tyr Ser
            1140                1145                1150

GTG AAG GAT AAG GAG GAC ACC CAG GTG GAC TCT GAG GCC CGA CCG ATG 3504
Val Lys Asp Lys Glu Asp Thr Gln Val Asp Ser Glu Ala Arg Pro Met
        1155                1160                1165
AAA GAT GAG ACC TTC GGC GAG TAC AGG TCC CTG GAG AGT GAC AAC GAG 3552
Lys Asp Glu Thr Phe Gly Glu Tyr Arg Ser Leu Glu Ser Asp Asn Glu
    1170                1175                1180
GAG AAG GCC TTT GGC AGC AGC CAG CCA TCG CTC AAC GGG GAC ATC AAG 3600
Glu Lys Ala Phe Gly Ser Ser Gln Pro Ser Leu Asn Gly Asp Ile Lys
1185                1190                1195                1200
CCC CTG GGC AGT GAC GAC AGC CTG GCC GAT TAT GGG GGC AGC GTG GAT 3648
Pro Leu Gly Ser Asp Asp Ser Leu Ala Asp Tyr Gly Gly Ser Val Asp
                1205                1210                1215
GTT CAG TTC AAC GAG GAT GGT TCG TTC ATT GGC CAG TAC AGT GGC AAG 3696
Val Gln Phe Asn Glu Asp Gly Ser Phe Ile Gly Gln Tyr Ser Gly Lys
            1220                1225                1230
AAG GAG AAG GAG GCG GCA GGG GGC AAT GAC AGC TCA GGG GCC ACT TCC 3744
Lys Glu Lys Glu Ala Ala Gly Gly Asn Asp Ser Ser Gly Ala Thr Ser
        1235                1240                1245
CCC ATC AAC CTT GCC GTG GCC CTA GAA TAG 3774
Pro Ile Asn Leu Ala Val Ala Leu Glu
    1250                1255

### SEQ ID NO: 19

SEQUENCE LENGTH: 25
SEQUENCE TYPE: Nucleic acid
STRANDNESS: Single
TOPOLOGY: Linear
MOLECULE TYPE: Synthetic DNA
SEQUENCE:

CCTCGAGATCTCAGCTGTCGACATG 25

### SEQ ID NO: 20

SEQUENCE LENGTH: 25
SEQUENCE TYPE: Nucleic acid
STRANDNESS: Single
TOPOLOGY: Linear
MOLECULE TYPE: Synthetic DNA
SEQUENCE:

TCGACAGCTGAGATCTCGAGGACGT 25

### SEQ ID NO: 21

SEQUENCE LENGTH: 32
SEQUENCE TYPE: Nucleic acid
STRANDNESS: Single
TOPOLOGY: Linear
MOLECULE TYPE: Synthetic DNA
SEQUENCE:

AATTCTGCCACCATGGTAGTGGTGCTGCGGTA 32

### SEQ ID NO: 22

SEQUENCE LENGTH: 32
SEQUENCE TYPE: Nucleic acid
STRANDNESS: Single
TOPOLOGY: Linear
MOLECULE TYPE: Synthetic DNA
SEQUENCE:

CACGTACCGCAGCACCACTACCATGGTGGCAG 32

### SEQ ID NO: 23

SEQUENCE LENGTH: 17
SEQUENCE TYPE: Nucleic acid
STRANDNESS: Single
TOPOLOGY: Linear
MOLECULE TYPE: Synthetic DNA
SEQUENCE:

CAGAGCTCCTACACGCAGTGGACCTG 17

### SEQ ID NO: 24

SEQUENCE LENGTH: 31
SEQUENCE TYPE: Nucleic acid
STRANDNESS: Single
TOPOLOGY: Linear
MOLECULE TYPE: Synthetic DNA
SEQUENCE:

TTCTAGACTAAGTGGCGAAGCCAGCAGGAGG 31

## Claims

1. A cDNA coding for a protein having the neuron-neuron cell adhesion activity of human neural cell adhesion molecule L1 wherein the neuron-neuron cell adhesion activity is responsible for homophilic binding between L1 molecules.

2. A gene comprising a cDNA inserted in plasmid HL1-N4 (FERM BP-3257), plasmid HL1-in3 (FERM BP-3256), plasmid HL1-NPCR3 (FERM BP-3377) or plasmid HL1-NPCR5 (FERM BP-3401).

3. A gene having
(1) a nucleotide sequence coding for the amino acid sequence shown in SEQ ID NO: 17 or 18; or
(2) a nucleotide sequence coding for an amino acid sequence modified by deletion, addition or replacement of one or more than one amino acid in SEQ ID NO: 17 or 18, while the modified amino acid sequence maintains the neuron-neuron cell adhesion activity of human neural cell adhesion molecule L1 responsible for homophilic binding between Li molecules; or
(3) a nucleotide sequence coding for polypeptide having the neuron-neuron cell adhesion activity of human neural cell adhesion molecule L1 responsible for homophilic binding between L1 molecules, and capable of hybridizing with the nucleotide sequence shown in SEQ ID NO: 17 or 18.

4. A gene comprising:
1) a nucleotide sequence coding for the amino acid sequence from the Met at position 1 to the Thr at position 1114 shown in SEQ ID NO: 17, or the amino acid sequence from the Met at position 1 to the Thr at position 1119 in SEQ ID NO: 18; or
(2) a nucleotide sequence coding for amino acid sequence modified by deletion, addition or replacement of one or more than one amino acid in the amino acid sequences defined in (1), while the modified amino acid sequence maintains the neuron-neuron cell adhesion activity of human neural cell adhesion molecule L1 responsible for homophilic binding between L1 molecules; or
(3) a nucleotide sequence coding for polypeptide having the neuron-neuron cell adhesion activity of human neural cell adhesion molecule L1 responsible for homophilic binding between L1 molecules, and capable of hybridizing with the nucleotide sequence defined in (1).

5. A protein having the neuron-neuron cell adhesion activity of human neural cell adhesion molecule L1 and having an amino acid sequence encoded by a gene according to claim 2, 3 (1) or 4 (1).

6. A protein according to claim 5 obtained by culturing a host transformed with an expression vector into which a gene according to claim 2, 3 (1) or 4 (1) has been inserted and recovering from the culture.

7. A protein according to claim 6, wherein the host is animal cells.

8. A process for production of a protein having the neuron-neuron cell adhesion activity of human neural cell adhesion molecule L1 characterized by culturing a host transformed with an expression vector containing a cDNA according to claim 1 or a gene according to any one of claims 2 to 4, and recovering a protein having an activity of human neural cell adhesion molecule L1 from the culture.

## Patentansprüche

1. cDNA, die ein Protein codiert, das die Neuron-Neuron-Zelladhäsionsaktivität des menschlichen Nervenzellen-Adhäsionsmoleküls L1 besitzt, wobei die Neuron-Neuron-Zelladhäsionsaktivität für die homophile Bindung zwischen L1-Molekülen verantwortlich ist.

2. Gen, umfassend eine cDNA, die in Plasmid HL1-N4 (FERM BP-3257), Plasmid HL1-in3 (FERM BP-3256), Plasmid HL1-NPCR3 (FERM BP-3377) oder Plasmid HL1-NPCR5 (FERM BP-3401) insertiert ist.

3. Gen mit
(1) einer Nucleotidsequenz, die die in Sequenz ID Nr. 17 oder 18 gezeigte Aminosäuresequenz codiert; oder
(2) einer Nucleotidsequenz, die eine Aminosäuresequenz codiert, die durch Deletion, Addition oder Ersetzen von einer oder mehr als einer Aminosäure in Sequenz ID Nr. 17 oder 18 modifiziert ist, wobei die modifizierte Aminosäuresequenz die Neuron-Neuron-Zelladhäsionsaktivität des menschlichen Nervenzellen-Adhäsionsmoleküls L1 bewahrt, das für die homophile Bindung zwischen L1-Molekülen verantwortlich ist; oder
(3) einer Nucleotidsequenz, die ein Polypeptid codiert, das die Neuron-Neuron-Zelladhäsionsaktivität des menschlichen Nervenzellen-Adhäsionsmoleküls L1 besitzt, das für die homophile Bindung zwischen L1-Molekülen verantwortlich ist, und zur Hybridisierung mit der in Sequenz ID Nr. 17 oder 18 gezeigten Nucleotidsequenz fähig ist.

4. Gen, umfassend:
(1) eine Nucleotidsequenz, die die Aminosäuresequenz vom Met an Position 1 bis zum Thr an Position 1114, wie in Sequenz ID Nr. 17 gezeigt, oder die Aminosäuresequenz vom Met in Position 1 bis zum Thr in Position 1119 in Sequenz ID Nr. 18 codiert; oder
(2) eine Nucleotidsequenz, die die Aminosäuresequenz codiert, die durch Deletion, Addition oder Ersetzen von einer oder mehr als einer Aminosäure in den Aminosäuresequenzen gemäß der Definition in (1) codiert, wobei die modifizierte Aminosäuresequenz die Neuron-Neuron-Zelladhäsionsaktivität von menschlichem Nervenzellen-Adhäsionsmolekül L1 bewahrt, das für die homophile Bindung zwischen L1-Molekülen verantwortlich ist; oder
(3) eine Nucleotidsequenz, die ein Polypeptid codiert, das die Neuron-Neuron-Zelladhäsionsaktivität vom menschlichen Nervenzellen-Adhäsionsmolekül L1 aufweist, das für die homophile Bindung zwischen L1-Molekülen verantwortlich ist und fähig ist zur Hybridisierung mit der in (1) definierten Nucleotidsequenz.

5. Protein mit der Neuron-Neuron-Zelladhäsionsaktivität des menschlichen Nervenzellen-Adhäsionsmoleküls L1 und mit einer Aminosäuresequenz, die durch ein Gen nach Anspruch 2, 3(1) oder 4 (1) codiert wird.

6. Protein nach Anspruch 5, erhalten durch Züchtung eines Wirts, der mit einem Expressionsvektor transformiert ist, in den ein Gen nach Anspruch 2, 3(1) oder 4(1) insertiert wurde, und Gewinnung aus der Kultur.

7. Protein nach Anspruch 6, wobei als Wirt tierische Zellen dienen.

8. Verfahren zur Herstellung eines Proteins mit der Neuron-Neuron-Zelladhäsionsaktivität vom menschlichen Nervenzellen-Adhäsionsmolekül L1, dadurch gekennzeichnet, daß man einen Wirt züchtet, der mit einem Expressionsvektor transformiert wurde, der eine cDNA nach Anspruch 1 oder ein Gen nach einem der Ansprüche 2 bis 4 enthält, und Gewinnung eines Proteins mit einer Aktivität des menschlichen Nervenzellen-Adhäsionsmoleküls L1 aus der Kultur.

## Revendications

1. ADNc codant pour une protéine ayant l'activité d'adhésion cellulaire neurone-neurone de la molécule L1 d'adhésion des cellules neuronales humaines dont l'activité d'adhésion cellulaire neurone-neurone est responsable de la liaison homophile entre molécules L1.

2. Gène comprenant un ADNc inséré dans le plasmide HL1-N4 (FERM BP-3257), le plasmide HL2-in3 (FERM BP-3256), le plasmide HL1-NPCR3 (FERM BP-3377) ou le plasmide H1-NPCR5 (FERM BP-3401).

3. Gène comprenant :
(1) une séquence de nucléotides codant pour la séquence d'acides aminés illustrée sous la référence SEQ ID N° 17 ou 18 ; ou
(2) une séquence de nucléotides codant pour une séquence d'acides aminés modifiée par délétion, addition, ou remplacement d'un ou de plusieurs des acides aminés de la séquence illustrée sous la référence SEQ ID N° 17 ou 18, la séquence d'acides aminés modifiée conservant l'activité d'adhésion cellulaire neurone-neurone de la molécule L1 d'adhésion des cellules neuronales humaines responsable de la liaison homophile entre molécules L1 ; ou
(3) une séquence de nucléotides codant pour un polypeptide ayant l'activité d'adhésion cellulaire neurone-neurone de la molécule L1 d'adhésion des cellules neuronales humaines responsable de la liaison homophile entre molécules L1, et capable de s'hybrider avec la séquence de nucléotides illustrée sous la référence SEQ ID N° 17 ou 18.

4. Gène comprenant :
(1) une séquence de nucléotides codant pour la séquence d'acides aminés allant de la Met en position 1 à la Thr en position 1114 illustrée sous la référence SEQ ID N° 17, ou la séquence d'acides aminés allant de la Met en position 1 à la Thr en position 1119 illustrée sous la référence SEQ ID N° 18 ; ou
(2) une séquence de nucléotides codant pour une séquence d'acides aminés modifiée par délétion, addition, ou remplacement d'un ou de plusieurs des acides aminés de la séquence d'acides aminés définie en (1), la séquence d'acides aminés modifiée conservant l'activité d'adhésion cellulaire neurone-neurone de la molécule L1 d'adhésion des cellules neuronales humaines responsable de la liaison homophile entre molécules L1 ; ou
(3) une séquence de nucléotides codant pour un polypeptide ayant l'activité d'adhésion cellulaire neurone-neurone de la molécule L1 d'adhésion des cellules neuronales humaines responsable de la liaison homophile entre molécules L1, et capable de s'hybrider avec la séquence de nucléotides définie en (1).

5. Protéine ayant l'activité d'adhésion cellulaire neurone-neurone de la molécule L1 d'adhésion des cellules neuronales humaines et ayant une séquence d'acides aminés codée par un gène selon la revendication 2, 3 (1) ou 4 (1).

6. Protéine selon la revendication 5, obtenue par mise en culture d'un hôte transformé par un vecteur d'expression dans lequel a été inséré un gène selon la revendication 2, 3 (1) ou 4 (1), et extraction de la culture.

7. Protéine selon la revendication 6, dans laquelle l'hôte est une cellule animale.

8. Procédé de production d'une protéine ayant l'activité d'adhésion cellulaire neurone-neurone de la molécule L1 d'adhésion des cellules neuronales humaines, caractérisé par la mise en culture d'un hôte transformé par un vecteur d'expression contenant un ADNc selon la revendication 1 ou un gène selon l'une quelconque des revendications 2 à 4, et extraction d'une protéine ayant une activité de la molécule L1 d'adhésion des cellules neuronales humaines à partir de la culture.
